# EUROPEAN PATENT SPECIFICATION

(11) **EP 1 345 589 B1**
(45) Date of publication and mention of the grant of the patent: **27.06.2007**
(21) Application number: 01998324.6
(22) Date of filing: 28.11.2001
(51) Int. Cl.: A61K 9/08

(54) **SOLVENT SYSTEMS FOR PHARMACEUTICAL AGENTS**
LÖSUNGSMITTELSYSTEME FÜR PHARMAZEUTISCHE MITTEL
SYSTEMES DE SOLVANTS POUR AGENTS PHARMACEUTIQUES

(30) Priority: 29.11.2000 US 253874 P
(43) Date of publication of application: 24.09.2003
(60) Divisional application: 07000689.5
(73) Proprietor: Lyotropic Therapeutics, Inc., Ashland, VA 23005 (US)
(72) Inventor: ANDERSON, David, M., Colonial Heights, VA 23834 (US)
(74) Representative: Lins, Edgar
(86) International application number: PCT/US2001/044287
(87) International publication number: WO 2002/043696

(56) References cited:
- US-A- 5 618 522
- US-A- 5 891 465

## Description

### BACKGROUND OF THE INVENTION

### Field of the Invention

The present invention relates to the solubilization of compounds which are difficult to solubilize. In particular, the invention provides compositions, solvent systems and methods for solubilizing such compounds.

### Background of the Invention

A significant number of compounds with potential pharmaceutical activity and application are poorly soluble in water. Of these, many are also difficult to solubilize with liquids and surfactants that are approved for use as, and appropriate for use as, excipients in pharmaceutical products. For example, for pharmaceutical actives where the most desirable format is the pill form for oral delivery, still the most common drug format by far, most liquid solvents and even surfactants will often be incompatible with the simplest tablet manufacturing procedures, since these procedures were generally developed with solids and powders in mind. Yet the application of these procedures to poorly-soluble drugs without the use of liquids or surfactants often yields a pill that achieves only a very limited bioavailability when administered. It should also be pointed out that while acidic (e.g., hydrochloride) or basic (e.g., sodium) salt forms of low-solubility drugs can often be soluble, such salts can precipitate in the body when they encounter pH conditions that deprotonate the acidic salt or protonate the basic salt.

US 5,891 465 provides compositions based on nutritional supplements that are encapsulated in Lipid vesicles for administration as an aerosol or liquid droplet spray.

For actives that are to be delivered by injection, solubilization of such compounds is made challenging by the very limited selection of liquids and surfactants that are approved for injection at levels that would be required to solubilize the drug. Water-miscible liquid excipients, most notably ethanol, are of limited value since, even when the drug is soluble in neat ethanol, it will often precipitate upon the addition of water, either diluent water for injection or in the aqueous milieu of body fluids, such as blood.

It would be highly desirable to have available composition, solvent systems, and methods for solubilizing compounds which are difficult to solubilize.

### SUMMARY OF THE INVENTION

The present invention provides compositions comprising a structured fluid and a compound present in the structured fluid, the compound being otherwise less than 5% by weight soluble in soybean oil. The structured fluid comprises a polar solvent, a lipid or a surfactant, and an essential oil or a dissolution/solubilization agent or both an essential oil and a dissolution/solubilization agent. The dissolution/solubilization may be: gentisic acid, benzoic acid, salicylic acid, N-alkylated amino acids, or a salt thereof; a fat-soluble vitamin or a salt thereof; an amphiphilic derivative of a water-soluble vitamin or a salt thereof; 8-hydroxyquinoline; or a low water-solubility amino acid or a salt thereof. The structured fluid is a reversed cubic liquid crystalline phase or a reversed hexagonal liquid crystalline phase.

The present invention further provides compositions comprising a structured fluid and a compound present in the structured fluid, the compound being otherwise less than 5% by weight soluble in soybean oil. The structured fluid comprises a polar solvent, a lipid or a surfactant, and an essential oil or a dissolution/solubilization agent or both an essential oil and a dissolution/solubilization agent. The dissolution/solubilization has at least one polar group in its molecular structure, a molecular weight from about 50 to about 500 Dalton and an octanol-water partition coefficient greater than about 10. The structured fluid is a reversed cubic liquid crystalline phase or a reversed hexagonal liquid crystalline phase.

The present invention further provides an internally administerable solvent system comprising a structured fluid formed from a polar solvent, a lipid or a surfactant, and an essential oil or a dissolution/solubilization agent or both an essential oil and a dissolution/solubilization agent. The dissolution/solubilization agent may be: gentisic acid, benzoic acid, salicylic acid, N-alkylated amino acids, or a salt thereof; a fat-soluble vitamin or a salt thereof; an amphiphilic derivative of a water-soluble vitamin or a salt thereof; 8-hydroxyquinoline; or a low water-solubility amino acid or a salt thereof. The structured fluid is a reversed cubic liquid crystalline phase or a reversed hexagonal liquid crystalline phase.

The present invention further provides an internally administerable solvent system comprising a structured fluid formed from a polar solvent, a lipid or a surfactant, and an essential oil or a dissolution/solubilization agent or both an essential oil and a dissolution/solubilization agent. The dissolution/solubilization agent has at least one polar group in its molecular structure, a molecular weight from about 50 to about 500 Dalton and an octanol-water partition coefficient greater than about 10.

The present invention further provides a method for solubilizing a compound, the compound being otherwise less than 5% by weight soluble in soybean oil. The method comprises the steps of combining the compound with a solvent system and allowing the compound to be incorporated into said solvent system. The solvent system comprises a structured fluid comprising a polar solvent, a lipid or a surfactant, and an essential oil or a dissolution/solubilization agent or both an essential oil and a dissolution/solubilization agent. The dissolution/solubilization may be: gentisic acid, benzoic acid, salicylic acid, N-alkylated amino acids, or a salt thereof; a fat-soluble vitamin or a salt thereof; an amphiphilic derivative of a water-soluble vitamin or a salt thereof; 8-hydroxyquinoline; or a low water-solubility amino acid or a salt thereof. The structured fluid is a reversed cubic liquid crystalline phase or a reversed hexagonal liquid crystalline phase.

### DESCRIPTION OF THE PREFERRED EMBODIMENTS

The present invention provides compositions, solvent systems and methods which are useful for solubilizing compounds that are otherwise difficult to solubilize (i.e. they are otherwise less than 5% by weight soluble in soybean oil). The compositions, solvent systems and methods of the present invention are based on the surprising discovery that certain compounds have a dramatic effect on altering the solubilizing properties of surfactant-water, and particularly lipid-water mixtures, i.e. the compounds act as dissolution/solubilization agents.

In order to facilitate understanding of the present invention, the following definitions and descriptions of terms utilized herein are provided:

### Definitions/Descriptions

**Dissolution**: Is meant that a compound under consideration is dissolving is undergoing dissolution.
**Solubilize**: Is meant to be essentially synonymous with the term "dissolve" or "dissolution", though with a different connotation; a compound under consideration is solubilized in a liquid or liquid crystalline material if and only if the molecules of the compound are able to diffuse within the liquid or liquid crystalline material as individual molecules, and that such material with the compound in it make up a single thermodynamic phase. It should be borne in mind that slightly different connotations are associated with the terms "dissolve" and "solubilize": typically the term "dissolve" is used to describe the simple act of putting a crystalline compound in a liquid or liquid crystalline material and allowing or encouraging that compound to break up and dissolve in the material, whereas the terms "solubilize" and "solubilization" generally refer to a concerted effort to find an appropriate liquid or liquid crystalline material that is capable of dissolving such compound.
**Matrix:** In the present context, a "matrix" is meant to be a material that serves as the host material for an active compound or compounds.
**Tunable:** In the present context, the solubilizing properties of a matrix can be said to be "tunable" if and only if the composition under consideration and/or structure of the matrix can be deliberately adjusted so as to substantially change the solubility of the active compound. **Difficultly-soluble:** In the present context, a compound (e.g., a pharmaceutical or nutritional active) can be said to be difficultly-soluble in water if a single therapeutic dose of the active requires more than about 100 ml of water or buffer to solubilize it; it can be said to be difficultly-soluble in oil if a single therapeutic dose of the active cannot be solubilized in less than about 10 ml of octanol; or if the compound is otherwise less than 5% by weight soluble in soybean oil. **Pharmaceutical active**: a compound or agent that exhibits biological activity, including nutritional, nutriceutical and/or pharmacological activity.
**Amphiphile:** an amphiphile can be defined as a compound that contains both a hydrophilic and a lipophilic group. *See* D. H. Everett, Pure and Applied Chemistry, vol. 31, no. 6, p. 611, 1972. It is important to note that not every amphiphile is a surfactant. For example, butanol is an amphiphile, since the butyl group is lipophilic and the hydroxyl group hydrophilic, but it is not a surfactant since it does not satisfy the definition, given below. There exist a great many amphiphilic molecules possessing functional groups which are highly polar and hydrated to a measurable degree, yet which fail to display surfactant behavior. *See* R. Laughlin, Advances in liquid crystals, vol. 3, p. 41, 1978.
**Surfactant**: A surfactant is an amphiphile that possesses two additional properties. First, it significantly modifies the interfacial physics of the aqueous phase (at not only the air-water but also the oil-water and solid-water interfaces) at unusually low concentrations compared to non-surfactants. Second, surfactant molecules associate reversibly with each other (and with numerous other molecules) to a highly exaggerated degree to form thermodynamically stable, macroscopically one-phase, solutions of aggregates or micelles. Micelles are typically composed of many surfactant molecules (10's to 1000's) and possess colloidal dimensions. See R. Laughlin, Advances in liquid crystals, vol. 3, p. 41, 1978. Lipids, and polar lipids in particular, often are considered as surfactants for the purposes of discussion herein, although the term 'lipid' is normally used to indicate that they belong to a subclass of surfactants which have slightly different characteristics than compounds which are normally called surfactants in everyday discussion. Two characteristics which frequently, though not always, are possessed by lipids are, first, they are often of biological origin, and second, they tend to be more soluble in oils and fats than in water. Indeed, many compounds referred to as lipids have extremely low solubilities in water, and thus the presence of a hydrophobic solvent may be necessary in order for the interfacial tension-reducing properties and reversible self-association to be most clearly evidenced, for lipids which are indeed surfactants. Thus, for example, such a compound will strongly reduce the interfacial tension between oil and water at low concentrations, even though extremely low solubility in water might make observation of surface tension reduction in the aqueous system difficult; similarly, the addition of a hydrophobic solvent to a lipid-water system might make the determination of self-association into nanostructured liquid phases and nanostructured liquid crystalline phases a much simpler matter, whereas difficulties associated with high temperatures might make this difficult in the lipid-water system.

Indeed, it has been in the study of nanostructured liquid crystalline structures that the commonality between what had previously been considered intrinsically different --'lipids' and 'surfactants' -- came to the forefront, and the two schools of study (lipids, coming from the biological side, and surfactants, coming from the more industrial side) came together as the same nanostructure observed in lipids as for surfactants. In addition, it also came to the forefront that certain synthetic surfactants such as dihexadecyldimethylammonium bromide which were entirely of synthetic, non-biological origin, showed 'lipid-like' behavior in that hydrophobic solvents were needed for convenient demonstration of their surfactancy. On the other end, certain lipids such as lysolipids, which are clearly of biological origin, display phase behavior more or less typical of water-soluble surfactants. Eventually, it became clear that for the purposes of discussing and comparing self-association and interfacial tension-reducing properties, a more meaningful distinction was between single-tailed and double-tailed compounds, where single-tailed generally implies water-soluble and double-tailed generally oil-soluble.

Thus, in the present context, any amphiphile which at very low concentrations lowers interfacial tensions between water and hydrophobe, whether the hydrophobe be air or oil, and which exhibits reversible self-association into nanostructured micellar, inverted micellar, or bicontinuous morphologies in water or oil or both, is a surfactant. The class of lipids simply includes a subclass of surfactants which are of biological origin.
**Lipid**: a lipid is considered to be a molecule formed by a hydrophilic moiety and a lipophilic moiety, the two linked together by bonds sufficiently flexible to yield a rather independent behavior. See Luzzati, in Biological Membranes, Chapter 3, page 72 (D. Chapman, ed. 1968). The terms "lipid" and "surfactant" are utilized interchangeably herein.
**Polar-apolar interface**: In a surfactant molecule, one can find a dividing point (or in some cases, 2 points, if there are polar groups at each end, or even more than two, as in Lipid A, which has seven acyl chains and thus seven dividing points per molecule) in the molecule that divide
the polar part of the molecule from the apolar part. In any nanostructured liquid phase or nanostructured liquid crystalline phase, the surfactant forms monolayer or bilayer films; in such a film, the locus of the dividing points of the molecules describes a surface that divides polar domains from apolar domains; this is called the "polar-apolar interface," or "polar-apolar dividing surface." For example, in the case of a spherical micelle, this surface would be approximated by a sphere lying inside the outer surface of the micelle, with the polar groups of the surfactant molecules outside the surface and apolar chains inside it. Care should be taken not to confuse this microscopic interface with macroscopic interfaces, separating two bulk phases, that are seen by the naked eye.
**Structured fluid:** Particularly useful mixtures from the point of view of microencapsulation and drug-delivery that occur in systems containing surfactant and polar solvents are structured fluids. For the purposes of this disclosure, a structured fluid is taken to be a fluid that has structural features on a length scale much larger than atomic dimensions, in particular fluids such as nanostructured liquids, nanostructured liquid crystals, and emulsions. Generally, structured fluids include L1, L2 and L3 phases, lyotropic liquid crystalline phases, emulsions, and microemulsions.

**L1 phase:** In an L1 phase that occurs in a system based on surfactants, the curvature of the polar-apolar interface is toward the apolar (non-polar) regions, generally resulting in particles -- normal micelles - that exist in a water-continuous medium. (Here "water" refers to any polar solvent). When these micelles transform from spherical to cylindrical as conditions or compositions change, they can start to fuse together and bicontinuity can result. In addition to the water continuity, the hydrophobic domains can connect up to form a sample-spanning network; this can still be an L1 phase. In addition, there are examples of L1 phases that show evidence of having no microstructure whatsoever. That is, there are no micelles, no well-defined domains, just surfactant molecules co-mingled in a structureless, one-phase liquid solution that is thus not a nanostructured material. These "structureless solutions" can sometimes be changed to nanostructured phases by simple change in composition without any phase change in between. In other words, thermodynamics does not dictate a phase boundary between a structureless solution and a nanostructured phase. This is, of course, in contrast with the case of a transition between a phase having long-range order (a liquid crystal or a crystal) and a phase lacking long-range order (a liquid), where a phase boundary is required by thermodynamics.

For L1 phases that occur in systems based on block copolymers, the terms 'polar' and 'apolar' may not apply, but in any case there are two (or in some cases more) domain types; we make the convention that the curvature of the A/B interface is toward A domains, so that a typical nanostructure would consist of particles, often sphere-like, of domain type A located in a continuum of B domains. As an example, in polystyrene-polyisoprene diblock copolymers, if the volume fraction of polystyrene blocks is very low, say 10%, then the usual microstructure will be polystyrene-rich spheres in a continuous polyisoprene matrix. Contrariwise, polyisoprene-rich spheres in a polystyrene-continuous matrix would be the likely structure for a 10% polyisoprene PS-PI diblock.

Identification of the nanostructured L1 phase. Since the L1 phase is a liquid phase, techniques have been developed to distinguished the nanostructured L1 phase from unstructured solution liquid phases. In addition to the experimental probes that are discussed below, there is a well-known body of knowledge that provides criteria by which one can determine a priori whether a given system should be expected to form nanostructured phases instead of simple unstructured solutions.

Since the formation of nanostructured liquid phases and nanostructured liquid crystalline phases is one requirement in the definition of a surfactant, in the discrimination of a nanostructured liquid from an unstructured solution it is extremely valuable to have criteria for determining whether a given compound is in fact a surfactant, criteria which provide for a number of tests for surfactancy in addition to methods discussed below for directly analyzing the liquid in question. A number of criteria have been discussed by Robert Laughlin in Advances in liquid crystals, 3:41, 1978. To begin with, Laughlin lists chemical criteria for determining *a priori* whether a given compound will be a surfactant, and this was discussed in detail above. If, based on these criteria, a compound is expected to be a true surfactant, then the compound is expected to form nanostructured phases in water. In addition, with such a compound in the presence of water and hydrophobe, nanostructured phases are also expected to form, normally incorporating at least a portion of the hydrophobe present.

In the event that a non-surfactant amphiphile is added to such a system, and in particular an amphiphilic organic solvent such as a short-chained alcohol, dioxane, tetrahydrofuran, dimethylformamide, acetonitrile, dimethylsulfoxide, etc., then structureless liquids could form, as the action of the organic solvent will generally be to disrupt colloidal aggregates and co-solubilize all the components.

Laughlin also goes on to discuss a number of criteria based on physical observations. One well-known criteria is the critical micelle concentration (CMC) which is observed in surface tension measurements. If the surface tension of an aqueous solution of the compound in question is plotted as a function of the concentration, then at very low concentrations, the surface tension will be seen to drop off sharply if the added compound is indeed a surfactant. Then, at a particular concentration known as the CMC, a sharp break will occur in this plot, as the slope of the line decreases drastically to the right of the CMC, so that the surface tension decreases much less with added surfactant. The reason is that above the CMC, added surfactant goes almost entirely into the creation of micelles, rather than to the air-water interface.

A second criterion tabulated by Laughlin is the liquid crystal criterion: if the compound forms liquid crystals at high concentrations, then it must be a surfactant and will form liquid crystalline phases at concentrations lower than those at which the occur. In particular, the L1 phase is usually found at concentrations of surfactant just lower than those that form normal hexagonal, or in some cases normal non-bicontinuous cubic, phase liquid crystals.

Another criterion discussed by Laughlin is based on the temperature differential between the upper limit of the Krafft boundary plateau and the melting point of the anhydrous compound. The Krafft boundary is a curve in the phase diagram of the binary system with compound and water; below the Krafft line are crystals, and above the Krafft line the crystals melt, so that there is a dramatic increase in solubility over a very narrow temperature range along the Krafft line. In the case of a true surfactant, this temperature differential is substantial: for example, in sodium palmitate, the melting point of the anhydrous compound is 288°C, while the Krafft line has its plateau at 69°C, so that the differential is 219°C. Laughlin goes on to discuss the case of dodecylamine, which has a temperature differential of 14 C, and has a small region in the phase diagram corresponding to liquid crystals, thus indicating a modest degree of association colloid behavior. In contrast, neither dodecylmethylamine nor dodecanol exhibit association behavior of the surfactant type, and both have zero temperature differential.

As in the case of liquid crystals, as discussed herein, given a material there are a number of experimental probes one can use to determine whether or not the material, in this case a liquid, is nanostructured, and these will be discussed in the context of the L1 phase, although they apply to all nanostructured liquids-- with the appropriate modifications. In such a determination, it is best to combine as many of these characterizations as feasible.

As with all the liquid phases, the L1 phase is optically isotropic in the absence of flow. It does not give a splitting in the ²H NMR bandshape with deuterated surfactant.

Also, in examination with crossed polarizing filters, the L1 phase of surfactant systems does not generally give birefringence even under moderate flow conditions. The situation with respect to birefringence in the case of block copolymer-based systems is complicated by the possibility of strain birefringence, so this is not a reliable method in that case.

Returning to the surfactant-based L1 phase, viscosity is generally quite low, considerably lower than any liquid crystals in the same system.

Using pulsed-gradient NMR to measure the effective self-diffusion coefficients of the various components, one finds that the self-diffusion of surfactant, and any added hydrophobe, is very low, typically on the order of 10⁻¹³ m²/sec or less (unless the phase is bicontinuous; see below.) This is because the primary means for diffusion of surfactant and hydrophobe is by diffusion of entire micelles, which is very slow. Also, the diffusion rates of surfactant and of hydrophobe should be nearly the same, for the same reason.

Small-angle x-ray scattering (SAXS) does not give sharp Bragg peaks in the nanometer range (nor any range), of course. However, analysis of the entire curve by several methods from the literature can give the length scale of the nanostructure. By analyzing the falloff of intensity at low wave numbers (but not too low compared to the inverse of the surfactant molecule length), one can determine the apparent radius of gyration: one plots intensity versus the square of the wave number, and takes the slope, to deduce Rg (the so-called Guinier plot.) The radius of gyration is then related to the dimensions of the micellar units by standard well-known formulae. This will fall in the range of nanometers. In addition, by plotting the product of intensity times the square of the wave number, versus the wave number -- the so-called 'Hosemann plot' -- one will find a peak that can also be related to the dimensions of the micelles; this has the advantage that it is less sensitive to interactions between micelles than is the radius of gyration.

For surfactant-based L1 phases which are bicontinuous, the above will change as follows. First, the viscosity can increase considerably when bicontinuity occurs, do to the rigidity of the surfactant film, which is continuous. Also, the self-diffusion rate of the surfactant and even of added hydrophobe (which can be deliberately added to a binary system as a marker) can increase dramatically, approaching or even exceeding the values in a lamellar phase in the same system. And while SAXS analyses, both the radius of gyration and the Hosemann plot, will give resulting dimensions in the nanometer range, these must be interpreted as characteristic length scales of the bicontinuous domain structure, rather than as dimensions of discrete particles. (In some models, such as the interconnected cylinders model of the author's thesis, or the Talmon-Prager model, a bicontinuous domain structure is represented as made up of units, which although seemingly 'particles', are in reality only building blocks for construction of a model bicontinuous geometry).

For L1 phases in block copolymer-based systems, this same SAXS analysis holds. In contrast, NMR bandshape and self-diffusion measurements in general do not carry over, nor do surface tension measurements. However, vapor transport measurements have been used in the past in place of NMR self-diffusion. In particular, if one can find a gas which is preferentially soluble in one of the domain types, but not in the other(s), then one can test for continuity of those domains by measuring the transport of that gas through the sample. If this is possible, then transport through the continuous domains (type B) in the micellar phase should be only slightly slower than that in the pure B polymer, whereas gas transport for a gas confined to A domains should be very low.

The shear modulus of a block copolymer-based micellar phase is determined largely by that of the polymer block forming the continuous domains, polymer B in our convention. Thus, for example, in a PS-PI diblock which is 10% PS, so that PS micelles form in a continuous PI matrix, the shear modulus would be close to that of pure polyisoprene, with only a slight increase due to the presence of the PS micelles. Interestingly, in the reverse case, with 90% PS and thus PI micelles in a continuous PS matrix, the elastomeric PI micelles can provide a shock-absorbing component which can improve the fracture characteristics over those of pure, glassy polystyrene.

**L2 phase**: This phase is the same as the L1 phase except that the roles of the polar region and the apolar region are reversed: the curvature of the polar-apolar interface is toward the polar domains, the interior of the micelles (if they exist) is water and/or other polar moieties, and the apolar domains (typically alkane chains of a lipid) form a continuous matrix -- although it is possible for the polar domains also to connect up to form a bicontinuous L2 phase. As above, this phase can be either nanostructured or structureless.

Identification of the nanostructured L2 phase. The guidelines for making an identification of the nanostructured L2 phase are the same as those given above for the L1 phase, with the following modifications. We need only discuss the surfactant-based L2 phase, since in the block copolymer-based systems the two types of micellar phases (A in B, and B in A) are equivalent, and above we discussed the identification of the micellar phase in block copolymer systems.

First, L2 phases are generally more prominent when the HLB is low, for example with ethoxylated alcohol surfactants having a small number of ethylene oxide groups (usually 5 or less, with typical alkyl chain lengths), or with double-chained surfactants. In terms of phase behavior, they generally occur at higher surfactant concentrations than even the reversed liquid crystalline phases; a location that is very common is for the L2 phase to border the reversed hexagonal phase at higher surfactant concentrations. For L2 phases which are not bicontinuous, it is the water self-diffusion which is very low, and measurement of the diffusion coefficient (by pulsed-gradient NMR, for example) should give a number on the order of 10⁻¹¹ m²/sec or less. Also, a Hosemann plot will give the size of the reversed micelles, which will essentially be the water domain size.
**L3 phase** (also known as the "anomolous phase):L2-phase regions in phase diagrams sometimes exhibit "tongues" sticking out of them: long, thin protrusions unlike the normal appearance of a simple L2 phase region. This sometimes appears also with some L1 regions, as described below. When one examines these closely, especially with X-ray and neutron scattering, they differ in a fundamental way from L2 phases. In an L2 phase, the surfactant film is generally in the form of a monolayer, with oil (apolar solvent) on one side and water (polar solvent) on the other. By contrast, in this nanostructured liquid "L3 phase," as these phases are called, the surfactant is in the form of a bilayer, with water (polar solvent) on both sides. The L3 phase is generally considered to be bicontinuous and, in fact, it shares another property with cubic phases: there are two distinct aqueous networks, interwoven but separated by the bilayer. So, the L3 phase is really very similar to the cubic phase, but lacking the long-range order of the cubic phase. L3 phases stemming from L2 phases and those stemming from L1 phases are given different names. "L3 phase" is used for those associated to L2 phases, and "L3* phase" for those associated to L1 phases.

Determination of the nanostructured L3 phase. Determination of the L3 phase in distinction to the other liquid phases discussed herein can be a sophisticated problem, requiring the combination of several analyses. The most important of these techniques are now discussed.

In spite of its optical isotropy when acquiescent and the fact that it is a liquid, the L3 phase can have the interesting property that it can exhibit flow birefringence. Often this is associated with fairly high viscosity, viscosity that can be considerably higher than that observed in the L 1 and L2 phases, and comparable to or higher than that in the lamellar phase. These properties are of course a result of the continuous bilayer film, which places large constraints on the topology, and the geometry, of the nanostructure. Thus, shear can result in the cooperative deformation (and resulting alignment) of large portions of the bilayer film, in contrast with, for example, a micellar L1 phase, where independent micellar units can simply displace with shear, displace with shear, and in any case a monolayer is generally much more deformable under shear than a bilayer. Support for this interpretation comes from the fact that the viscosity of L3 phases is typically a linear function of the volume fraction of surfactant. Snabre, P. and Porte, G. (1990) Europhys. Lett. 13:641.

Sophisticated light, neutron, and x-ray scattering methodologies have been developed for determination of nanostructured L3 phases. Safinya, C.R., Roux, D., Smith, G.S., Sinha, S.K., Dimon, P., Clark, N.A. and Bellocq, A.M. (1986) Phys. Rev. Lett. 57:2718; Roux, D. and Safinya, C.R. (1988) J. Phys. France 49:307; Nallet, F., Roux, D. and Prost, J. (1989) J. Phys. France 50:3147. The analysis of Roux, et al., in Roux, D., Cates, M.E., Olsson, U., Ball, R.C., Nallet, F. and Bellocq, A.M., Europhys. Lett. purportedly is able to determine that the nanostructure has two aqueous networks, separated by the surfactant bilayer, which gives rise to a certain symmetry due to the equivalence of the two networks.

Fortunately, determination of the nanostructured nature of an L3 phase based on phase behavior can be more secure than in the case of typical L1, L2 or even microemulsion phases. This is first of all because the L3 phase is often obtained by addition of a small amount (a few percent) of oil or other compound to a lamellar or bicontinuous cubic phase, or small increase of temperature to these same phases. Since these liquid crystalline phases are easy to demonstrate to be nanostructured (Bragg peaks in X-ray, in particular), one can be confident that the liquid phase is also nanostructured when it is so close in composition to a liquid crystalline phase. After all, it would be extremely unlikely that the addition of a few percent of oil to a nanostructured liquid crystalline phase would convert the liquid crystal to a structureless liquid. Indeed, pulsed-gradient NMR self-diffusion measurements in the Aerosol OT - brine system show that the self-diffusion behavior in the L3 phase extrapolates very clearly to those in the nearby reversed bicontinuous cubic phase. This same L3 phase has been the subject of a combined SANS, self-diffusion, and freeze-fracture electron microscopy study. Strey, R., Jahn, W., Skouri, M., Porte, G., Marignan, J. and Olsson, U., in "Structure and Dynamics of Supramolecular Aggregates," S.H. Chen, J.S. Huang and P. Tartaglia, Eds., Kluwer Academic Publishers, The Netherlands. Indeed, in SANS and SAXS scattering analysis of L3 phases, a broad interference peak is often observed at wave vectors that correspond to d-spacings that are the same order of magnitude as those in bicontinuous cubic phases that are nearby in the phase diagram, and the author has developed a model for L3 phase nanostructure which is an extrapolation of known structures for bicontinuous cubic phases. Anderson, D.M., Wennerström, H. and Olsson, U. (1989) J. Phys. Chem. 93:4532.

**Microemulsion**: A microemulsion may be defined as a thermodynamically stable, low viscosity, optically isotropic, microstructured liquid phase containing oil (apolar liquid), water (polar liquid), and surfactant. *See also* Danielsson, I. and Lindman, B. (1981) Colloids and Surfaces, 3:391. Thermodynamically stable liquid mixtures of surfactant, water and oil are usually referred to as microemulsions. While being macroscopically homogeneous, they are structured on a microscopic length scale (10-1,000 Angstrom) into aqueous and oleic microdomains separated by a surfactant-rich film. *See* Skurtveit, R. and Olsson, U. (1991) J. Phys. Chem. 95:5353. A key defining feature of a microemulsion is that it contain an "oil" (apolar solvent or liquid), in addition to water and surfactant; it is always microstructured by definition. In general, because of the strong tendency for oil and water to phase segregate, in the absence of an organic solvent capable of co-solubilizing oil and water (such as ethanol, THF, dioxane, DMF, acetonitrile, dimethylsulfoxide, and a few others), a clear, single-phase liquid containing oil, water and surfactant must be a microemulsion, and one can safely conclude on that basis alone that the phase is nanostructured. Note that a microemulsion can also be an L1 or L2 phase, especially if it contains well-defined micelles; however, if it is an L1 phase, then the micelles are necessarily swollen with oil. The microemulsion is a nanostructured liquid phase. If a liquid with "oil," water and surfactant has a characteristic domain size larger than the nanometer range, that is, in the micron range, then it is no longer a microemulsion but rather a "miniemulsion" or plain emulsion; both of the latter are non-equilibrium. The term microemulsion was introduced, despite the fact that L1 and L2 phases can contain oil, and can even be bicontinuous, because it is fairly common for three-component oil-water-surfactant/lipid systems to evolve continuously from water-continuous to bicontinuous to oil-continuous with no phase boundaries in between. In this case, it does not make sense to try to set a dividing point between the "L1" and "L2" regions of the phase diagram; so instead, one just refers to the whole region as "microemulsion" -- recognizing that at the high-water-content end of this region the structure is that of an oil-swollen L1 phase, and at the high-oil-content end of this region the structure is that of an L2 phase. (In terms of Venn diagrams, there are overlaps between microemulsions and L1 and L2 phases, though not between L1 and L2 phases.) As discussed below, the microstructure of microemulsions is quite generally describable in terms of a monolayer film of surfactant that divides oil-rich domains from water-rich domains. This surfactant/lipid-rich dividing film can enclose to form micelles, or connect up into a network structure to form a bicontinuous microemulsion.

It must be pointed out that an emulsion is not a nanostructured liquid, as the term is applied herein. To begin with, the characteristic length scale in an emulsion, which essentially is the average size of an emulsion droplet, is generally much larger than the characteristic length scale in a nanostructured liquid, and falls in the range of microns instead of nanometers. While recent efforts to produce emulsions with submicron droplet sizes have given rise to smaller-droplet emulsions and to the advent of the term "mini emulsion," there remain crucial differences which exclude emulsions and mini emulsions from the realm of microemulsions. The nanostructured liquid phases described herein, including microemulsions, exist at thermodynamic equilibrium, in contrast to emulsions which are not equilibrium phases but only metastable materials. Furthermore, a nanostructured liquid which is acquiescent and fully equilibrated is optically transparent, whereas an emulsion is generally opaque -- ordinary milk is an emulsion, for example.

Determination of nanostructured microemulsions. The methods and guidelines discussed above for determination of nanostructured L1 phases carry over to the determination of nanostructured microemulsion phases, with the following variations.

For microemulsions which do not clearly fall under either the L1 phase or the L2 phase descriptions -- which is the remaining case to be treated here -- we take note that many, if not most, of these are bicontinuous, and in the context of a single liquid phase containing oil, water and surfactant, bicontinuity provides strong proof that the phase is nanostructured, since emulsions and other common liquids are never bicontinuous. This issue has been addressed in "On the demonstration of bicontinuous structures in microemulsions," Lindman, B., Shinoda, K., Olsson, U., Anderson, D. M., Karlstrom, G. and Wennerstrom, H. (1989) Colloids and Surfaces 38:205. The time-tested way to demonstrate bicontinuity is to use pulsed-gradient NMR, and measure the effective self-diffusion coefficients of both oil and water separately; generally it is best to measure also the self-diffusion of the surfactant. Electrical conductivity can also be used to establish water continuity, although this is prone to problems associated with "hopping" processes. Fluoresence quenching has also been used for continuity determination. Sanchez-Rubio, M., Santos-Vidals, L. M., Rushforth, D. S. and Puig, J. E. (1985) J. Phys. Chem. 89:411. Small-angle neutron and x-ray scattering analyses have been used to examine bicontinuity. Auvray. L., Cotton, R., Ober, R. and Taupin, J. (1954) J. Phys. Chem. 88:4586. Porod analysis of SAXS curves has been used to deduce the presence of interfaces, thus proving that a nanostructure is present. Martino, A. and Kaler, E.W. (1990) J. Phys. Chem. 94:1627. Freeze-fracture electron microscopy, with extremely fast rates of freezing, has been used to study microemulsions, and is the result of decades of development on fixation methods for nanostructured liquids; a critical review discussing the methods and the reliability of the results has been given. Talmon, Y., in K.L. Mittal and P. Bothorel (Eds), Vol. 6, Plenum Press, New York, 1986, p. 1581.

In the event that an oil-water-surfactant liquid phase is not clearly an L1 or L2 phase, and does not show strong evidence of bicontinuity, then the analysis to demonstrate that it is nanostructured can be fairly involved, and no single technique will suffice. In general, one would apply the measurements discussed in this section, such as SANS or SAXS, NMR self-diffusion, cryo EM, etc., to attempt to rationalize the data within the context of a model nanostructure.

**Emulsions**: An emulsion has been defined as a dispersion of drops of some liquid in another immiscible one, which exhibits more or less stability depending on the application [J.-L. Salager in Pharmaceutical Emulsions and Suspensions, eds. F. Nielloud and G. Marti-Mestres, from the series Drugs and the Pharmaceutical Sciences, Vol. 105, Marcel Dekker, NY, NY (2000), pp. 19-72]. In most emulsions the dispersed droplets are stabilized by the presence of surfactant localized at the surface of the droplets, which is in fact the polar-apolar interface, often referred to as the "oil-water" interface. Simple emulsions are classified as oil-in-water, or o/w, or water-in-oil, w/o, emulsions, and double or multiple emulsions as o/w/o or w/o/w. A "biemulsion" is yet another type of emulsion in which two distinct types of oil droplets are dispersed in the same continuous polar (or "water") phase. In contrast with microemulsions, which by definition are thermodynamically stable, all emulsions are metastable and will eventually settle into two (or more) phases, and are thus often referred to as two-phase systems.

While a simplistic view of emulsion stabilization, in which a monolayer of surfactant is present at the droplet surface, is a popular concept, the situation at least in many cases-and perhaps quite generally-may involve the presence of nanostructured lyotropic liquid crystalline phases at the droplet surface. According to model of the structure of emulsions advanced by Stig Friberg [cf. Larsson, K., and S. Friberg, Eds. 1990, Food Emulsions, 2nd Edition, Marcel Dekker, Inc. NY], lamellar liquid crystalline or, commonly, lamellar crystalline coatings stabilize the oil droplets in an oil-in-water emulsion, and the water droplets in a water-in-oil emulsion.

Other reports have been published in which nanostructured lyotropic liquid crystals are implicated in stabilizing emulsion droplets, and liquid crystalline phases other than lamellar have also been described. An undecylglycerylether-modified silicone surfactant was found to form emulsions in the region where a reversed hexagonal phase was in equilibrium with excess water and oil phases [Nagatani N, Fukuda K, Suzuki T., J. Colloid Interface Sci. 2001 Feb 15;234(2):337-343]. The present author has observed hardy emulsions in the soy phosphatidcylcholine-benzene-water system, at ratios of benzene:PC of about 0.8:1 and with excess water, where a reversed discrete cubic phase is in equilibrium with excess water. Other authors have seen stable emulsions where cubic phases are implicated as the stabilizing layer [Rodriguez C, Shigeta K, Kunieda H., J. Colloid Interface Sci. 2000 Mar 15;223(2):197-204]. In one carefully studied system, emulsion stability was seen to increase substantially at a concentration where liquid crystalline phases appeared [Vaziri A, Warburton B., J. Microencapsul 1995 Jan-Feb;12(1):1-5]. Viscous liquid crystalline phases often referred to as "gels" have been reported to yield high-stability emulsions when found in equilibrium with excess "oil" and "water" phases [Ali AA, Mulley BA, J. Pharm. Pharmacol. 1978 Apr;30(4):205-13].

**Lyotropic liquid crystalline phases.** Lyotropic liquid crystalline phases include the normal hexagonal, normal bicontinuous cubic, normal discrete cubic, lamellar, reversed hexagonal, reversed bicontinuous cubic, and reversed discrete cubic liquid crystalline phases, together with the less well-established normal and reversed intermediate liquid crystalline phases.

The nanostructured liquid crystalline phases are characterized by domain structures, composed of domains of at least a first type and a second type (and in some cases three or even more types of domains) having the following properties:
a) the chemical moieties in the first type domains are incompatible with those in the second type domains (and in general, each pair of different domain types are mutually incompatible) such that they do not mix under the given conditions but rather remain as separate domains; (for example, the first type domains could be composed substantially of polar moieties such as water and lipid head groups, while the second type domains could be composed substantially of apolar moieties such as hydrocarbon chains; or, first type domains could be polystyrene-rich, while second type domains are polyisoprene-rich, and third type domains are polyvinylpyrrolidone-rich);
b) the atomic ordering within each domain is liquid-like rather than solid-like, lacking lattice-ordering of the atoms; (this would be evidenced by an absence of sharp Bragg peak reflections in wide-angle x-ray diffraction);
c) the smallest dimension (e.g., thickness in the case of layers, diameter in the case of cylinders or spheres) of substantially all domains is in the range of nanometers (viz., from about 1 to about 100 nm); and
d) the organization of the domains conforms to a lattice, which may be one-, two-, or three-dimensional, and which has a lattice parameter (or unit cell size) in the nanometer range (viz., from about 5 to about 200 nm); the organization of domains thus conforms to one of the 230 space groups tabulated in the International Tables of Crystallography, and would be evidenced in a well-designed small-angle x-ray scattering (SAXS) measurement by the presence of sharp Bragg reflections with d-spacings of the lowest order reflections being in the range of 3-200 nm.

### Lamellar phase: The lamellar phase is characterized by:

1. Small-angle x-ray shows peaks indexing as 1:2:3:4:5:... in wave number.
2. To the unaided eye, the phase is either transparent or exhibits mild or moderate turbidity.
3. In the polarizing optical microscope, the phase is birefringent, and the well-known textures have been well described by Rosevear, and by Winsor (e.g., Chem. Rev. 1968, p.1). The three most pronounced textures are the "Maltese crosses," the "mosaic" pattern, and the "oily streaks" patterns. The Maltese cross is a superposition of two dark bands (interference fringes) roughly perpendicular to each other, over a roughly circular patch of light (birefringence), forming a distinctive pattern reminiscent of the WW1 German military symbol. The variations on this texture, as well as its source, is thoroughly described in J. Bellare, Ph.D. Thesis, Univ. of Minnesota, 1987. The "mosaic" texture can be envisioned as the result of tightly packing together a dense array of deformed Maltese crosses, yielding dark and bright patches randomly quilted together. The "oily streaks" pattern is typically seen when the (low viscosity) lamellar phase flows between glass and cover slip; in this pattern, long curved lines are seen, upon close inspection under magnification (e.g., 400x), to be composed of tiny striations which run roughly perpendicular to the line of the curve, as ties make up a railroad track (to be contrasted with the hexagonal texture discussion below). In some cases, particularly if the phase is massaged gently between glass and cover slip for a period of time, the lamellar phase will align with its optic axis parallel to the line of sight in the microscope, resulting in a disappearance of the birefringence.

For lamellar phases in surfactant-water systems:
1. viscosity is low, enough so that the material flows (e.g., when a tube containing the phase is tipped upside down).
2. the self-diffusion rates of all components are high, comparable to their values in bulk-e.g., the effective self-diffusion coefficient of water in the lamellar phase is comparable to that in pure water. Since the surfactants that form liquid crystals are usually not liquid at ambient temperatures, the reference point for the self-diffusion coefficient of the surfactant is not clear-cut, and in fact, the effective (measured) self-diffusion coefficient of the surfactant in the lamellar phase is often taken to be the reference point for interpreting measurements in other phases.
3. if the surfactant is deuterated in the head group, and the ²H NMR bandshape measured, one finds two spikes with the splitting between them twice what it is in the hexagonal phase.
4. In terms of phase behavior, the lamellar phase generally occurs at high surfactant concentrations in single-tailed surfactant / water systems, typically above 70% surfactant; in double-tailed surfactants, it often occurs at lower concentrations, often extending well below 50%. It generally extends to considerably higher temperatures than do any other liquid crystalline phases that happen to occur in the phase diagram.

For lamellar phases in single-component block copolymer systems:
1. shear modulus is generally lower than other liquid crystalline phases in the same system.
2. in terms of phase behavior, the lamellar phase generally occurs at volume fractions of the two blocks is roughly 50:50.

### Normal hexagonal phase: The normal hexagonal phase is characterized by:

1. Small-angle x-ray shows peaks indexing as 1:√3:2: √7:3 ... ; in general, √ (h 2 + hk + k²), where h and k are integers -- the Miller indices of the two-dimensional symmetry group.
2. To the unaided eye, the phase is generally transparent when fully equilibrated, and thus often considerably clearer than any nearby lamellar phase.
3. In the polarizing optical microscope, the phase is birefringent, and the well-known textures have been well described by Rosevear, and by Winsor (e.g., Chem. Rev. 1968, p.1). The most distinctive of these is the "fan-like" texture. This texture appears to be made up of patches of birefringence, where within a given patch, fine striations fan out giving an appearance reminiscent of an oriental fan. Fan directions in adjacent patches are randomly oriented with respect to each other. A key difference distinguishing between lamellar and hexagonal patterns is that the striations in the hexagonal phase do not, upon close examination at high magnification, prove to be composed of finer striations running perpendicular to the direction of the larger striation, as they do in the lamellar phase.

For normal hexagonal phases in surfactant-water systems:
1. viscosity is moderate, more viscous than the lamellar phase but far less viscous than typical cubic phases (which have viscosities in the millions of centipoise).
2. the self-diffusion coefficient of the surfactant is slow compared to that in the lamellar phase; that of water is comparable to that in bulk water.
3. the ²H NMR bandshape using deuterated surfactant shows a splitting, which is one-half the splitting observed for the lamellar phase.
4. in terms of phase behavior, the normal hexagonal phase generally occurs at moderate surfactant concentrations in single-tailed surfactant / water systems, typically on the order of 50% surfactant. Usually the normal hexagonal phase region is adjacent to the micellar (L1) phase region, although non-bicontinuous cubic phases can sometimes occur in between. In double-tailed surfactants, it generally does not occur at all in the binary surfactant-water system.

For hexagonal phases in single-component block copolymer systems, the terms "normal" and "reversed" do not generally apply (although in the case where one block is polar and the other apolar, these qualifiers could be applied in principle). The shear modulus in such a hexagonal phase is generally higher than a lamellar phase, and lower than a bicontinuous cubic phase, in the same system. In terms of phase behavior, the hexagonal phases generally occurs at volume fractions of the two blocks on the order of 35:65. Typically, two hexagonal phases will straddle the lamellar phase, with, in each case, the minority component being inside the cylinders (this description replacing the 'normal/reversed' nomenclature of surfactant systems).
Reversed hexagonal phase: In surfactant-water systems, the identification of the reversed hexagonal phase differs from the above identification of the normal hexagonal phase in only two respects:
1. The viscosity of the reversed hexagonal phase is generally quite high, higher than a typical normal hexagonal phase, and approaching that of a reversed cubic phase. And,
2. In terms of phase behavior, the reversed hexagonal phase generally occurs at high surfactant concentrations in double-tailed surfactant / water systems, often extending to, or close to, 100% surfactant. Usually the reversed hexagonal phase region is adjacent to the lamellar phase region which occurs at lower surfactant concentration, although bicontinuous reversed cubic phases often occur in between. The reversed hexagonal phase does appear, somewhat surprisingly, in a number of binary systems with single-tailed surfactants, such as those of many monoglycerides (including glycerol monooleate), and a number of nonionic PEG-based surfactants with low HLB.

As stated above in the discussion of normal hexagonal phases, the distinction between 'normal' and 'reversed' hexagonal phases makes sense only in surfactant systems, and generally not in single-component block copolymer hexagonal phases.

### Nonnal bicontinuous cubic phase: The normal bicontinuous cubic phase is characterized by:

1. Small-angle x-ray shows peaks indexing to a three-dimensional space group with a cubic aspect. The most commonly encountered space groups, along with their indexings, are: Ia3d (#230), with indexing √6: √8: √14:4: ... ; Pn3m (#224), with indexing √2: √3:2: √6: √8: ... ; and Im3m (#229), with indexing √2: √4: √6: √8: √10 :....
2. To the unaided eye, the phase is generally transparent when fully equilibrated, and thus often considerably clearer than any nearby lamellar phase.
3. In the polarizing optical microscope, the phase is non-birefringent, and therefore there are no optical textures.

For normal bicontinuous cubic phases in surfactant-water systems:
1. viscosity is high, much more viscous than the lamellar phase and even more viscous than typical normal hexagonal phases. Most cubic phase have viscosities in the millions of centipoise.
2. no splitting is observed in the NMR bandshape, only a single peak corresponding to isotropic motion.
3. in terms of phase behavior, the normal bicontinuous cubic phase generally occurs at fairly high surfactant concentrations in single-tailed surfactant / water systems, typically on the order of 70% surfactant with ionic surfactants. Usually the normal bicontinuous cubic phase region is between lamellar and normal hexagonal phase regions, which along with its high viscosity and non-birefringence make its determination fairly simple. In double-tailed surfactants, it generally does not occur at all in the binary surfactant-water system.

For bicontinuous cubic phases in single-component block copolymer systems, the terms "normal" and "reversed" do not generally apply (although in the case where one block is polar and the other apolar, these qualifiers could be applied in principle). The shear modulus in such a bicontinuous cubic phase is generally much higher than a lamellar phase, and significantly than a hexagonal phase, in the same system. In terms of phase behavior, the bicontinuous cubic phases generally occur at volume fractions of the two blocks on the order of 26:74. In some cases, two bicontinuous cubic phases will straddle the lamellar phase, with, in each case, the minority component being inside the cylinders (this description replacing the'normal/reversed' nomenclature of surfactant systems), and hexagonal phases straddling the cubic-lamellar-cubic progression.

### Reversed bicontinuous cubic phase: The reversed bicontinuous cubic phase is characterized by:

In surfactant-water systems, the identification of the reversed bicontinuous cubic phase differs from the above identification of the normal bicontinuous cubic phase in only one respect. In terms of phase behavior, the reversed bicontinuous cubic phase is found between the lamellar phase and the reversed hexagonal phase, whereas the normal is found between the lamellar and normal hexagonal phases; one must therefore make reference to the discussion above for distinguishing normal hexagonal from reversed hexagonal. A good rule is that if the cubic phase lies to higher water concentrations than the lamellar phase, then it is normal, whereas if it lies to higher surfactant concentrations than the lamellar then it is reversed. The reversed cubic phase generally occurs at high surfactant concentrations in double-tailed surfactant / water systems, although this is often complicated by the fact that the reversed cubic phase may only be found in the presence of added hydrophobe ('oil') or amphiphile. The reversed bicontinuous cubic phase does appear in a number of binary systems with single-tailed surfactants, such as those of many monoglycerides (include glycerol monooleate), and a number of nonionic PEG-based surfactants with low HLB.

It should also be noted that in reversed bicontinuous cubic phases, though not in normal, the space group #212 has been observed. This phase is derived from that of space group #230. As stated above in the discussion of normal bicontinuous cubic phases, the distinction between 'normal' and 'reversed' bicontinuous cubic phases makes sense only in surfactant systems, and generally not in single-component block copolymer bicontinuous cubic phases.

### Normal discrete (non-bicontinuous) cubic phase: The normal non-bicontinuous cubic phase is characterized by:

1. Small-angle x-ray shows peaks indexing to a three-dimensional space group with a cubic aspect. The most commonly encountered space group in surfactant systems is Pm3n (#223), with indexing √2: √4: √5 : .... In single-component block copolymers, the commonly observed space group is Im3m, corresponding to body-centered, sphere-packings, with indexing √2: √4: √6: √8: ...
2. To the unaided eye, the phase is generally transparent when fully equilibrated, and thus often considerably clearer than any associated lamellar phase.
3. In the polarizing optical microscope, the phase is non-birefringent, and therefore there are no optical textures.

For normal discrete cubic phases in surfactant-water systems:
1. viscosity is high, much more viscous than the lamellar phase and even more viscous than typical normal hexagonal phases. Most cubic phase have viscosities in the millions of centipoise, whether discrete or bicontinuous.
2. also in common with the bicontinuous cubic phases, there is no splitting in the NMR bandshape, only a single isotropic peak.
3. in terms of phase behavior, the normal discrete cubic phase generally occurs at fairly low surfactant concentrations in single-tailed surfactant / water systems, typically on the order of 40% surfactant with ionic surfactants. Usually the normal discrete cubic phase region is between normal micellar and normal hexagonal phase regions, which along with its high viscosity and non-birefringence make its determination fairly simple. In double-tailed surfactants, it generally does not occur at all in the binary surfactant-water system.

For discrete cubic phases in single-component block copolymer systems, the terms "normal" and "reversed" do not generally apply (although in the case where one block is polar and the other apolar, these qualifiers could be applied in principle). The shear modulus in such a discrete cubic phase is generally dependent almost entirely on the shear modulus of the polymer that forms the blocks in the continuous phase. In terms of phase behavior, the discrete cubic phases generally occur at very low volume fractions of one or other of the two blocks, on the order of 20% or less.

### Reversed discrete cubic phase: The reversed discrete cubic phase is characterized by:

In surfactant-water systems, the identification of the reversed discrete cubic phase differs from the above identification of the normal discrete cubic phase in three respects:
1. In terms of phase behavior, the reversed discrete cubic phase if found between the lamellar phase and the reversed hexagonal phase, whereas the normal is found between the lamellar and normal hexagonal phases; one must therefore make reference to the discussion above for distinguishing normal hexagonal from reversed hexagonal. A good rule is that if the cubic phase lies to higher water concentrations than the lamellar phase, then it is normal, whereas if it lies to higher surfactant concentrations than the lamellar then it is reversed. The reversed cubic phase generally occurs at high surfactant concentrations in double-tailed surfactant / water systems, although this is often complicated by the fact that the reversed cubic phase may only be found in the presence of added hydrophobe ('oil') or amphiphile. The reversed discrete cubic phase does appear in a number of binary systems with single-tailed surfactants, such as those of many monoglycerides (include glycerol monooleate), and a number of nonionic PEG-based surfactants with low HLB.
2. The space group observed is usually Fd3m, #227.
3. The self-diffusion of the water is very low, while that of any hydrophobe present is high; that of the surfactant is generally fairly high, comparable to that in the lamellar phase.

As stated above in the discussion of normal discrete cubic phases, the distinction between 'normal' and 'reversed' discrete cubic phases makes sense only in surfactant systems, and generally not in single-component block copolymer discrete cubic phases.
Intermediate phases: These phases occur quite rarely, and when they are found they generally occupy very narrow regions in the phase diagram. Presently the structures of many of these are unknown or under debate. The intermediate phases can be classified as follows:

Normal int(1) phases occur at lower surfactant concentration than the normal bicontinuous cubic phase, adjacent to the hexagonal phase. Viscosity is generally low or moderately low, no higher than that of the normal hexagonal phase. The phase is birefringent, with textures typically similar to those of the hexagonal phase. Self-diffusion of the components is very similar to those in the hexagonal phase. Small-angle x-ray shows a lower-symmetry space group than the cubic phases, typically monoclinic. Fairly sophisticated NMR bandshape and SAXS analyses can be used to distinguish this phase from the normal hexagonal phase. See Henriksson, U., Blackmore, E.S., Tiddy, G.J.T. and Soderman, O. (1992) J. Phys. Chem. 96:3894. Typically bandshape splittings will be intermediate between those of hexagonal and the zero splitting of the isotropic phase, which provides good evidence of an intermediate phase.

Normal int(2) is found at higher concentrations than the normal bicontinuous cubic phase, adjacent to the lamellar phase. These bear close resemblance, both in terms of property and probably also in terms of structure, to the normal bicontinuous cubic phases, except that they are birefringent, and show differences in NMR bandshape and SAXS analyses. Optical textures are somewhat unusual, in some cases resembling lamellar textures and in some resembling hexagonal, but these can be considerably coarser than either of the more common phases. As in the int(1) phases, the space group is of lower symmetry, typically rhombohedral or tetragonal, requiring two unit cell parameters for characterization, and making SAXS analysis difficult. In general, if the squares of the d-spacing ratios cannot be fit to a simple integral scheme, then an intermediate phase structure is suspect.

Reversed int(2) is found at lower concentrations than the reversed bicontinuous cubic phase, adjacent to the lamellar phase. These are birefringent, and show unusual in NMR bandshape and SAXS analyses. As in the int(1) and the int(2) phases, the space group is of lower symmetry, typically rhombohedral or tetragonal, requiring two unit cell parameters for characterization, and making SAXS analysis difficult, through the presence of Bragg peaks in the SAXS spectrum which do not index to a cubic or hexagonal lattice (which have only one lattice parameter) is, together with optical birefringence, indication of an intermediate phase. Space groups which are likely for bicontinuous intermediate phases have been discussed in a publication by the present author. D. M. Anderson, supplement to J. Physique, Proceedings of Workshop on Geometry and Interfaces, Aussois, France, Sept. 1990, C7-1- C7-18.

Of particular value for drug-delivery are those phases that can exist in equilibrium with excess dilute aqueous solution, so that they can maintain their integrity in body fluids, at least to the point where solubilized actives remain solubilized and do not suffer appreciable precipitation. These "insoluble" phases can be operationally divided into the lamellar phases, and the reversed liquid crystalline phases.

Those lamellar phases that can co-exist with a dilute aqueous solution can in many cases be formulated into liposomes, which are comprised essentially of one or more bilayers wrapped up into a sphere-like. (most commonly) shape enclosing a volume of water. In cases where one or more active compounds (e.g., drugs) are to be incorporated into the lipid bilayer region (as opposed to into the aqueous core), the solubilization techniques of the present invention can often be very utilitarian.

The reversed liquid crystalline phases can be formulated into particles of several types of potentially great importance in drug-delivery.

In addition, all of the liquid crystalline phases are of potential use in their bulk (i.e., non-microparticulate) form, as "gel-like" materials for topical application, implantation, ingestion, or, when their viscosity permits, parenteral, subcutaneous or intraperitoneal administration. Again in these cases, the solubilization techniques of the present invention can often be very useful.
**Polar solvents.** The polar solvents employed in the practice of the present invention include but are not limited to:
a. water;
b. glycerol;
c. ethylene glycol or propylene glycol;
d. ethylammonium nitrate;
e. one of the acetamide series: acetamide, N-methyl acetamide, or dimethylacetamide;
f. low-molecular weight polyethylene glycol (PEG);
g. a mixture of two or more of the above.

Especially preferred polar solvents are water, glycerol, ethylene glycol, dimethylacetamide, and polyethylene glycol.

### Compounds that are insoluble in both water and lipid.

It is a mistake to tacitly assume that a compound that is water-insoluble should be soluble in lipid--in other words, that the terms "hydrophobic" and "lipophilic" are equivalent. It is true that when a water-insoluble molecule can be fairly cleanly divided into a very small number (generally 3 or less) of well-defined polar and apolar regions, then the compound is often soluble in lipid. However, particularly in the world of pharmaceutical actives, it is common to find a larger number of polar and apolar groups dispersed in a single molecule. In such cases, one strategy for solubilizing the drug in a lipid bilayer system is to introduce lipid-soluble compounds that contain polar, and especially charged, groups, as is the case with salts of gentisic acid and related acids, and ascorbyl palmitate.

For example, consider the structure of dantrolene. As one moves along the length of the molecular structure diagram of dantrolene, one finds: a polar group (nitro group), low-polarity group (aromatic ring), moderately-polar group (furanyl ring), polar group (methylamino), and finally a hydantoin group which is charged or uncharged depending on pH. This compound has a solubility of approximately 150 mg/L in water, and even its sodium salt has a solubility on the order of 300 mg/L. Further, its solubility in simple phospholipid-water systems is also very low, too low to be of practical pharmaceutical importance. It is difficult to imagine a configuration of the drug in a lipid bilayer that would avoid direct contact between at least one of the polar groups with an acyl chain of the phospholipid.

The case of paclitaxel is even more demonstrative of molecules that cannot be neatly divided into polar and apolar sections. The molecule has 47 carbon atoms, includes 3 distinct aromatic rings, and has an exceedingly low solubility in water. However, a significant number of polar groups are present: one amide group, 3 hydroxyls, 4 ester bonds, another carbonyl group, and an cyclopropoxy ring.

Table 1 lists representative pharmaceutical compounds from some of the major therapeutic categories which are of low solubility in water (a fraction of a percent solubility), and tabulates the number of polar groups on the molecule. The table demonstrates that many, if not most, water-insoluble drugs contain at least 3 polar groups, and would be expected to have low solubility in a simple lipid-water mixture. The incorporation of a dissolution/solubilization agent in accordance with the present invention remedies this. Examination of the chemical structure of each of these compounds furthermore reveals that the polar groups are spread throughout the molecule, so that only in rare cases would the molecule be able to situate itself in a simple (lipid-water) bilayer with an orientation analogous to that of a surfactant. Most of these drugs listed are also problematic when attempts are made to solubilize the drug in water by converting the drug to a salt, such as a hydrochloride, or sodium salt for example; for example, some would precipitate at the pH of the body milieu, others would decompose, etc.

| TABLE 1 | | | | | | | | | | |
|---|---|---|---|---|---|---|---|---|---|---|
| Therapeutic Category | Compound | A | B | C | D | E | F | G | H | Total |
| | | | | | | | | | | |
| ACE inhibitor | Enalapril | 1 | | 1 | 1 | 1 | | | | 4 |
| beta-Adrenergic agonist | Albuterol | 1 | 2 | | | | 1 | | | 4 |
| beta-Adrenergic blocker | Sulfinalol | 1 | 1 | | | | 1 | | 2 | 5 |
| Anabolic | Nandrolone | | 1 | | | 1 | | | | 2 |
| Analgesic (narcotic) | Morphine | 1 | 1 | | | | 1 | | 1 | 4 |
| Analgesic (non-narcotic) | Aspirin | | | 1 | | 1 | | | | 2 |
| Androgen | Testosterone | | 1 | | | 1 | | | | 2 |
| Anesthetic (intravenous) | Hexobarbitol | | | | 2 | 1 | | | | 3 |
| Anorexic | Cyclexedrine | 1 | | | | | | | | 1 |
| Anthelmintic (cestodes) | Niclosamide | | | | 1 | | 1 | | 1 | 3 |
| Anthelmintic (nematodes) | Mebendazole | 2 | | | 1 | 1 | | | | 4 |
| Anthelmintic (schistosoma) | Amphotalide | 1 | | | 1 | 1 | | | 1 | 4 |
| Antiacne | Retinoic acid | | | 1 | | | | | | 1 |
| Antiamebic | Emetine | 1 | | | | | | | 4 | 5 |
| Antianginal | Nifedipine | 1 | | | | 2 | | | 1 | 4 |
| Antiarrhythmic | Quinidine | 2 | 1 | | | | | | 1 | 4 |
| Antibiotic (amphenicol) | Chloramphenicol | | 1 | | 1 | | | | 1 | 3 |
| Antibiotic (ansamycin) | Rifamide | | 2 | | 2 | 3 | 2 | | 4 | 13 |
| Antibiotic (lactam) | Ampicillin | 1 | | 1 | 2 | | | | 1 | 5 |
| Antibiotic (macrolide) | Erythromycin A | 1 | 5 | | | 2 | | | 4 | 12 |
| Antibiotic (tetracycline) | Tetracycline | 1 | 4 | | 1 | 2 | 1 | | | 9 |
| Antibacterial (quinolone) | Ciprofloxacin | 3 | | 1 | | 1 | | | | 5 |
| Antibacterial (sulfonamide) | Sulfamoxole | 2 | | | | | | | 2 | 4 |
| Antibacterial (sulfone) | Dapsone | 2 | | | | | | | 1 | 3 |
| Anticholinergic | Atropine | 1 | 1 | | | 1 | | | | 3 |
| Anticoagulant | Warfarin | | 1 | | | 2 | | | | 3 |
| Anticonvulsant | Nitrazapem | 1 | | | 1 | | | | 1 | 3 |
| Antidepressant | Zometapine | 4 | | | | | | | | 4 |
| Antidiabetic | Glyburide | | | | 3 | | | | 2 | 5 |
| Antidiarrheal | Uzarin | | 7 | | | 1 | | | 1 | 9 |
| Anti-inflammatory | Aspirin | | | 1 | | 1 | | | | 2 |
| Antineoplastic | Taxol | | 3 | | 1 | 5 | | | 1 | 10 |
| Antineoplastic | Etiposide | | 2 | | | 1 | 1 | | 8 | 12 |
| Skeletal muscle relaxant | Dantrolene | 1 | | | 2 | | | | 2 | 5 |

| | | | | | | | | | | |
|---|---|---|---|---|---|---|---|---|---|---|
| A= amino; B= hydroxyl; C= carboxyl; D=amide; E= carbonyl; F= phenolic; G= cation H= other | | | | | | | | | | |

Table 2 also lists candidate pharmaceutical agents for use in the present invention.

| TABLE 2 | | |
|---|---|---|
| Pharm Class | Generic Name | Trade Name |
| | | |
| Anabolic steroid | Nandrolone decanoate | Androlone |
| Analgesic | Fentanyl citrate | Sublimaze |
| Androgen | Testosterone | Testoderm, etc |
| Anthelmintic | Albendazole | Albenza |
| Antibiotic, antineoplastic | Doxorubicin | Rubex |
| Antibiotic, antineoplastic | Epirubicin | Ellence |
| Antibiotic, antineoplastic | Idarubicin | Idamycin |
| Antibiotic, antineoplastic | Valrubicin | Valstar |
| Anticholinergic | Oxybutinin | Ditropan |
| Antifungal | Amphotericin B | Fungizone, etc. |
| Antihypertensive | Enalaprilat | Vasotec |
| Antimitotic | Docetaxel | Taxotere |
| Antimitotic | Paclitaxel | Taxol |
| Antimitotic | Vinblastine | Velban |
| Antimitotic | Vincristine | Oncovin |
| Antimitotic | Vinorelbine | Navelbine |
| Antineoplastic | Batimastat | |
| Antiplatelet | Eptifibatide | Integrilin |
| Antiplatelet | Tirofiban | Aggrastat |
| Antipsychotic, anesthetic | Droperidol | Droperidol, Inapsine |
| Antiviral | Acyclovir | Zovirex; Valtrex |
| Antiviral | Pentafuside | none |
| Antiviral | Saquinavir | Fortovase |
| Asthma anti-inflammatory | Cromolyn | Intal |
| CNS stimulant | Doxapram | Dopram |
| DNA topoisomerase inhibitor | SN-38 (Irinotecan) | Camptosar |
| DNA topoisomerase inhibitor | Topotecan | Hycamtin |
| Enzyme inhibitor | Hemin | Panhematin |
| Epipodophyllotoxin | Daunorubicin | Daunorubicin; DaunoXome* |
| Epipodophyllotoxin | Teniposide | Vumon |
| Folate antagonist | Trimetrexate | Neutrexin |
| Gastric antisecretory | Octreotride | Sandostatin |
| Hormone | Leuprolide | Lupron, Viadur |
| Immunosuppressant | Clyclosporin A | Sandimmune |
| Inotropic agent | Milrinone lactate | Primacor |
| Narcotic agonist/antagonist | Buprenorphine | Buprenex |
| Narcotic agonist/antagonist | Nalbuphine | Nubain |
| Platinum complex | Carboplatin | Paraplatin |
| Platinum complex | Cisplatin | PlatinolAQ |
| Platinum complex | Mitoxantrone | Novantrone |
| Sex hormone | Estradiol | Kestrone, etc. |
| Sex hormone | Hydroxyprogesterone | Hylutin |
| Thyroid hormone | L-Thyroxine | Levothroid, etc. |
| TNF inhibitor (arthritis) | Etanercept | Enbrel |
| Urinary cholinergic | Neostigmine | Prostigmin |
| Vasodilator | Epoprostenol | Flolan |

In addition, the following pharmaceutical actives may be considered for inclusion in the present invention:

### ANTINEOPLASTICS

### Alkylating Agents

Alkyl Sulfonates - Busulfan, Improsulfan, Piposulfan,
*Aziridines -* Benzodepa, Carboquone, Meturedepa, Uredepa;
Antibiotics - Carubicin, Carzinophilin, Daunorubicin, 6-Diazo-5-oxo-L-norieucine, Doxorubicin, Epirubicin, Mitomycins, Mycophenolic Acid, Tubercidin, Ubenimex, Zorubicin;

### Antimetabolites

Folic Acid Analogs - Denopterin, Pteropterin, Trimetrexate;
Purine Analogs - Fludarabine, 6-Mercaptopurine, Thiamiprine;
Pyrimidine Analogs - Ancitabine, Azacitidine, 6-Azauridine, Carmofur, Cytarabine, Doxifluridine, Enocitabine, Floxuridine, Fluorouracil, Tegafur;
Others - Aceglatone, Amsacrine, Bestrabucil, Bisantrene, Carboplatin, Cisplatin, Demecolcine, Diaziquone, Eflornithine, Etoglucid, Etoposide, Lentinan, Lonidamine, Mitoguazone, Mitoxantrone, Mopidamol, Phenamet, Pirarubicin, Podophyllinic Acid, Razoxane, Sizofiran, Spirogermanium, Taxol, Teniposide, Tenuazonic Acid, Triaziquone, Vinblastine, Vincristine, Vindesine;

### Antineoplastic (Hormonal)

Androgens - Calusterone, Dromostanolone Propionate, Epitiostanol, Mepitiostane, Testolactone, Antiadrenals - Mitotane, Trilostane;
Andandrogens - Flutamide, Nilutamide;
Antiestrogens - Tamoxifen, Toremifene;
Estrogens - Fosfestrol, Hexestrol, Polyestradiol Phosphate;
LH-RH Analogs - Buserelin, Goserelin, Leuprolide, Triptorelin;
Progestogens - Chlormadinone Acetate, Medroxyprogesterone, Megestrol Acetate, Melengestrol;

### Antineoplastic (Radiation Source)

¹³¹I-Ethiodized Oil;

### Antineoplastic Adjuncts

Folic Acid Replenisher - Folinic Acid,
Uroprotective - Mesna.

The present invention provides for a range of lipid-based solubilization systems of reversed hexagonal and reversed cubic phase mixtures, whose solubilization properties can be tuned over a broad range. The property that is of importance in the solubilization of actives that have low solubilities in both water and simple lipid-water mixtures is recognized in the present invention to be the concentration and type of polar groups preferentially located in the lipid bilayer or at the polar-apolar interface.

Herein, a pharmaceutical active is taken to be of low water-solubility if a therapeutic dose of the active requires more than about 100 ml of water to solubilize it. Similarly, in the present invention a pharmaceutical active is taken to be of low lipid-solubility if a therapeutic dose of the active requires more than about 10 ml octanol in order to solubilize it. The choice of octanol is a natural one since it is the standard solvent in the definition of the important octanol-water partition coefficient, K_{ow}. Further, a compound is considered to be of low lipid-solubility if it is less than 5% by weight soluble in soybean oil.

Compounds with aromaticity, i.e., the presence of aromatic rings, unsaturation, i.e., the presence of carbon-carbon double bonds, and/or polar groups in their molecular structure and with molecular weights in the range of from about 50 to 500 Dalton, and preferably from about 100 to about 200 Daltons and fairly high oil-water partition coefficients (generally greater than about 10, preferably greater than about 100 and more preferably greater than about 1000) can fulfill the desired functions of a dissolution/solubilization agent of the present invention. Desirable compounds for use as dissolution/solubilization agents of the present invention have a molecular structure that contains a number of polar groups, such as, for example, a phenolic group, a carboxyl group, a primary alcohol group, and an amino group, and yet partitions to a significant extent into the lipid bilayer of a lipid-water system. It is believed that this is due to the presence of aliphatic and aromatic hydrocarbon groups. This results in a significant concentration of polar groups in the bilayer, penetrating into the bilayer from the polar-apolar interface. (In a microstructured liquid or liquid crystal containing films of surfactant, the polar-apolar interface is generally taken to be described by an envisioned mathematical surface passing through the loci of dividing points, one on each surfactant molecule, separating the polar group from the apolar portion of the molecule, such that on one side of the surface the microenvironment is predominantly polar and on the other side of the surface the microenvironment is predominantly apolar.) The presence of such polar groups affects the properties of the microenvironment in the hydrophobic portion of the bilayer, and this can result in greatly increased solubility of compounds that are both lipid-insoluble and water-insoluble. Examples of polar groups are as follows:
aldehyde, ketone, carboxylic ester, carboxylic acid, isocyanate, amide, acyl cyanoguanidine, acyl guanylurea, acyl biuret, N,N-dimethylamide, nitrosoalkane, nitroalkane, nitrate ester, nitrite ester, nitrone, nitrosamine, pyridine N-oxide, nitrile, isonitrile, amine borane, amine haloborane, sulfone, phosphine sulfide, arsine sulfide, sulfonamide, sulfonamide methylimine, alcohol (monofunctional), ester (monofunctional), secondary amine, tertiary amine, mercaptan, thioether, primary phosphine, secondary phosphine, and tertiary phosphine.

Some polar groups which are operative as surfactant head groups, and thus, for example, an alkane chain linked to one of these polar groups would be expected to form nanostructured liquid and liquid crystalline phases, are:
a. Anionics: carboxylate (soap), sulfate, sulfamate, sulfonate, thiosulfate, sulfinate, phosphate, phosphonate, phosphinate, nitroamide, tris(alkylsulfonyl)methide, xanthate;
b. Cationics: ammonium, pyridinium, phosphonium, sulfonium, sulfoxonium;
c. Zwitterionics: ammonio acetate, phosphoniopropane sulfonate, pyridinioethyl sulfate;
d. Semipolars: amine oxide, phosphoryl, phosphine oxide, arsine oxide, sulfoxide, sulfoximine, sulfone diimine, ammonio amidate.

Laughlin also demonstrates that as a general rule, if the enthalpy of formation of a 1:1 association complex of a given polar group with phenol (a hydrogen bonding donor) is less than 5 kcal, then the polar group will not be operative as a surfactant head group.

In addition to solubilizing drugs that are otherwise difficult to solubilize, the dissolution/solubilization agents and approaches disclosed in herein can also serve another important role, that of providing a solubilizing matrix into which the pharmaceutically active compound partitions preferentially over water or body fluid (e.g., blood, etc.). For example, certain drugs are not poorly water soluble, yet are more effective in certain situations when they are solubilized in a hydrophobic or amphiphilic environment, as opposed to solubilized in water. In particular, solubilization in a more hydrophobic environment can yield sustained release, or targeted release by holding on to the drug until the matrix reaches the correct site or environment, and/or provide a protective milieu for the drug, or more generally provide a local microenvironment with more favorable chemical or physical properties for production, storage, or application.

As an example, Experiment 10 reported herein, the local anesthetic bupivicaine is solubilized-in its low-solubility, free base form-in a liquid crystal incorporating an essential oil as solubilizing agent, in spite of the fact that the more frequently used hydrochloride salt is water soluble. This liquid crystal formulation with the free base form so solubilized provides an evironment into which the bupivicaine partitions strongly, since the value of K_{ow} is approximately 1500. This provides an encapsulation approach in which the drug will remain in the matrix even when the processing of the matrix involves contact with excess water, and furthermore will provide for sustained release of the anesthetic, which in the water-solubilized hydrochloride form has a therapeutic half life of only a few hours.

Desirable dissolution/solubilization agents are gentisic acid ethanolamine and its relatives, alpha-tocopherol and its relatives, ascorbyl palmitate and its relatives, and 8-hydroxyquinoline and tryptophan. Additional desirable dissolution/solubilization agents are essential oils and their components.

### Gentisic acid ethanolamine and its relatives.

Gentisic acid ethanolamine is a molecule that contains a number of polar groups--a phenolic group, a carboxyl group, a primary alcohol group, and an amino group--and yet partitions to a significant extent into the lipid bilayer of a lipid-water system. It is believed that this is due to the presence of aliphatic and aromatic hydrocarbon groups. This results in a significant concentration of polar groups in the bilayer, penetrating into the bilayer from the polar-apolar interface. The presence of such groups affects the properties of the microenvironment in the hydrophobic portion of the bilayer, and this can result in greatly increased solubility of compounds that are both lipid-insoluble and water-insoluble.

Relatives of gentisic acids are those acids which contain an acidic group covalently bonded-possibly through a spacer group-to an aromatic ring, wherein each of these two groups (the acidic group and the aromatic group) makes up at least 5% of the molecular weight of the molecule, also including salts formed by reacting such acids with bases, particularly sodium, potassium, ammonium, calcium, magnesium, ferrous, zinc, aluminum, and bismuth salts of these acids.

This excipient is used as an antioxidant in certain existing formulations, and in particular is used at levels of 100 mg per injection in a multi-vitamin injectable product, M.V.I.-12^{™}, indicating a high level of safety and low-toxicity associated with the compound. The compound is a salt. However, when incorporated into lipid-water systems, the compound affects the lipid bilayer properties by introducing the polar groups cited above into the bilayer, or at least in very close proximity to the polar-apolar interface.

When gentisic acid ethanolamine and/or one of its relatives is incorporated into systems containing lipid and an essential oil or one or more component(s) of an essential oil, the essential oil assists in the solubilization of the gentisic salt, and the gentisic salt can then work synergistically with the essential oil in solubilizing difficult actives.

Other compounds with aromatic rings and polar groups and with molecular weights in the range of from about 50 to about 500 Daltons, and preferably from about 100 to about 200 Daltons and fairly high oil-water partition coefficients (generally greater than about 10, preferably greater than about 100 and more preferably greater than about 1000) can fulfill the same functions as gentisic acid and its salts in the present context. Benzoic acid, salicylic acid, acetylsalicylic acid, N-acetyltryptophan, other N-alkylated amino acids, and derivatives of these compounds fall into this category. In addition to ethanolamine, other low-toxicity bases that can be reacted with these acids include tromethamine (tris[hydroxyethyl]methylamine), diethanolamine, ammonia, diethylamine, guanidine, 8-hydroxyquinoline, and ethylenediamine.

### Alpha-tocopherol and its relatives.

Alpha-tocopherol, or vitamin E, is a liquid of very low solubility in water and very high solubility in lipid bilayers with unsaturated-chain lipids. Being a long-chain compound with a hydroxyl group at one end of the molecule, the hydroxyl group is strongly preferentially located at the polar-apolar interface. It is also known that alpha-tocopherol interacts strongly with unsaturated bonds in phospholipid bilayers, having a major effect on the fluidity of the bilayer. These properties mean that alpha-tocopherol has effects in increasing the degree of order in the bilayer, relative to the normal disordering effect of simple saturated alcohols that are liquids at ambient temperatures. Superposed on this effect is the effect of increasing the curvature of the substituent monolayers toward water, and the net effect of these factors is that addition of alpha-tocopherol to lamellar phases in phosphatidylcholine-water systems tends to induce transitions to reversed hexagonal and cubic liquid crystalline phases, and with surprisingly high melting points for these reversed liquid crystalline phases. In addition to the advantages that this yields in terms of the usefulness of reversed liquid crystalline phases, this high melting point often permits the incorporation of actives, or solubilizers of actives, at higher concentrations than would be possible otherwise.

For example, when alpha-tocopherol and/or its relatives are used in liquid crystalline phase-based formulations incorporating essential oils or components thereof, the alpha-tocopherol can enable the use of the essential oil by reversing the effect of essential oils in melting liquid crystals. Thus, alpha-tocopherol typically allows much higher ratios of essential oil to lipid than would be possible without the tocopherol, and this increased ratio translates into greater solubilization of drug.

Alpha-tocopherol is an extremely low toxicity compound, which has a long history of use in nutritional and pharmaceutical products. For example, Lentaron^{™} 250 mg solution for parenteral usage is one example of a pharmaceutical formulation in which alpha-tocopherol is used in an injectable formulation.

### Ascorbyl palmitate.

This amphiphilic derivative of Vitamin C (which has Vitamin C activity) can serve two important functions in lipid-based solubilization systems. First, it possesses effective polar groups that can modulate the microenvironment of the lipid bilayer, especially increasing the concentration of polar groups near the polar-apolar interface of the lipid bilayer, and more especially on the apolar side of that interface. These polar groups are the same groups that make vitamin C itself (ascorbic acid) one of the most water-soluble organic solid compounds available: ascorbic acid is soluble to about 30 wt/wt% in water (very close to the solubility of sodium chloride, for example), and is highly soluble even as a salt with a multivalent ion such as calcium or ferrous. And second, when the pH increases so as to convert a fraction of the ascorbyl palmitate to a more soluble salt, such as sodium ascorbyl palmitate, then this converted fraction is a true surfactant, with a 16-carbon saturated chain, that frequently has the effect of raising the melting point of liquid crystalline phases.

### 8-Hydroxyquinoline.

As with ascorbyl palmitate, this compound can both increase the concentration of polar groups (phenolic groups and amino groups) in a lipid bilayer, and the melting point of liquid crystalline phases. Both of these effects can increase the likelihood that a liquid crystalline phase can be found which will incorporate a water-insoluble, lipid-insoluble active compound at appreciable concentration.

### Essential oils and components thereof.

Essential oils from plant sources (by "essential oils" we man essential oils, their extracts and components, and mixtures thereof, as more fully described later) comprise a rather large and chemically diverse group of liquids that include many low-toxicity oils and components. The term "essential oils" is intended to include:
allspice berry, amber essence, anise seed, arnica, balsam of peru, basil, bay, bay leaf, bergamot, bois de rose (rosewood), cajeput, calendula (marigold pot), white camphor, caraway seed, cardamon, carrot seed, cedarwood, celery, german or hungarian chamomile, roman or english chamomile, cinnamon, citronella, clary sage, clovebud, coriander, cumin, cypress, eucalyptus, fennel, siberian fir needle, frankincense (olibanum oil), garlic, rose geranium, ginger, grapefruit, hyssop, jasmine, jojoba, juniper berry, lavender, lemon, lemongrass, lime, marjoram, mugwort, mullein flower, myrrh gum, bigarade neroli, nutmeg, bitter orange, sweet orange, oregano palmarosa, patchouly, pennyroyal, black pepper, peppermint, petite grain, pine needle, poke root, rose absolute, rosehip seed, rosemary, sage, dalmation sage, santalwood oil, sassafras, spearmint, spikenard, spruce (hemlock), tangerine, tea tree, thuja (cedar leaf), thyme, vanilla extract, vetivert, wintergreen, witch hazel (hamamelia) extract, or ylang ylang (cananga) extract.

Those essential oils which have a strong tendency to form reversed hexagonal and bicontinuous cubic phases together with common insoluble lipids and water, at oil:lipid ratios between approximately 1:2 and 1:1, are preferred for applications involving reversed liquid crystals, and include: ylang ylang, clovebud, cedarwood, spearmint, ginger, patchouli, santalwood, carrot seed, fir needle, peppermint and mixtures of peppermint and thyme.

Of these essential oils, those which are labeled as GRAS (Generally Regarded As Safe) for certain modes of application are particularly preferred, and include:
ylang ylang, clovebud, spearmint, ginger, patchouly , sandalwood, carrot seed, peppermint and mixtures of peppermint and thyme.

The following are examples of components of essential oils:
2,6-dimethyl-2,4,6-octatriene; 4-propenylanisole; benzyl-3-phenylpropenoic acid; 1,7,7-trimethylbicyclo[2.2.1]heptan-2-ol; 2,2-dimethyl-3-methylenebicyclo[2.2.1]heptane;
1,7,7-trimethylbicyclo[2.2.1]heptane; trans-8-methyl-n-vanillyl-6-nonenamide; 2,2,5-trimethylbicyclo[4.1.0]hept-5-ene; 5-isopropyl-2-methylphenol; p-mentha-6,8-dien-2-ol;
p-mentha-6,8-dien-2-one; .beta.-caryophyllene; 3-phenylpropenaldehyde; mixed geranial and neral; 3,7-dimethyl-6-octenal; 3,7-dimethyl-6-octen-1-ol; 4-allylanisole; ethyl 3-phenylpropenoic acid; 3-ethoxy-4-hydroxybenzaldehyde; 1,8-cineole; 4-allyl-2-methoxyphenol; 3,7,11-trimethyl-2,6,10-dodecatrien-1-ol; 1,3,3-trimethylbicyclo[2.2.1]heptan-2-ol; 1,3,3-trimethylbicyclo[2.2.1]heptan-2-one; trans-3,7-dimethyl-2,6-octadien-1-ol; trans-3,7-dimethyl-2,6-octadien-1-yl acetate; 3-methyl-2-(2-pentenyl)-2-cyclopenten-1-one; p-mentha-1,8-diene; 3,7-dimethyl-1,6-octadien-3-ol; 3,7-dimethyl-1,6-octadien-3-yl acetate; p-menthan-3-ol; p-menthan-3-one; methyl 2-aminobenzoate; methyl-3-oxo-2-(2-pentenyl)-cyclopentane acetate; methyl 2-hydroxybenzoate; 7-methyl-3-methylene-1,6-octadiene; cis-3,7-dimethyl-2,6-octadien-1-ol; 2,6,6-trimethylbicyclo[3.1.1]hept-2-ene; 6,6-dimethyl-2-methylenebicyclo[3.1.1]heptane; p-menth-4(8)-en-3-one; p-menth-1-en-4-ol; p-mentha-1,3-diene; p-menth-1-en-8-ol; 2-isopropyl-5-methylphenol.

A good many of these oils are GRAS, Generally Regarded As Safe, by the FDA. When essential oils are mixed with lipid (or surfactant) and polar solvent, at ratios between about 1:2 and about 1.5:1, most preferably between 0.7:1 and about 1.2:1, the lipid-rich phase is generally either liquid, or liquid crystalline. It has been found that a number of GRAS essential oils tend to form liquid crystalline phases under these conditions, and these include:
peppermint;
spearmint;
sweet basil;
thyme;
ginger;
rosemary;
fennel;
sage;
jasmine; and
clove.
Thus, for example, when phosphatidylcholine is mixed with water and one of these aforesaid oils at a phosphatidylcholine:oil:water ratio of about 42:34:24, liquid crystals generally result at ambient temperature (depending on the source and purity of the oil). Those GRAS oils which tend to liquify lipid-water mixtures include:
marjoram;
palmarosa; and
bois de rose (rosewood).
Oil of bay and oil of vanilla are borderline between these two.

The ability of a number of these GRAS oils to solubilize difficultly-soluble drugs has been demonstrated by solubilizing paclitaxel in the oils. Here are some selected results:

| Essential oil | Paclitaxel solubility (w/w, approx) |
|---|---|
| Oil of bay | 16% |
| Peppermint | >5% |
| Thyme | 10% |
| Sweet basil | 7% |
| Palmarosa | >10% |

In addition to these GRAS oils, several non-GRAS oils are good solvents for paclitaxel, including in particular sandalwood oil. Although non-GRAS, sandalwood oil exhibits low toxicity in animal studies, including studies where parenteral routes are used. Paclitaxel is soluble in sandalwood oil to a level of at least 5%.
**Fat-soluble vitamins and salts thereof**. Vitamins A, D, E and K in many of their various forms and provitamin forms are considered as fat-soluble vitamins, and in addition to these a number of other vitamins and vitamin sources or close relatives are also fat-soluble and have polar groups, relatively high octanol-water partition coefficients, and molecular weights between about 50 and 500. Clearly, the general class of such compounds has a history of safe use and high benefit to risk ratio, making them of potential use as excipients and potentially as functional excipients. These compounds as well as salts thereof can be particularly useful as dissolution/solubilization agents in the present invention, especially in modulating the polarity properties of the surfactant-rich films present in the structured fluid as described herein. According to the Merck Index (Eleventh Edition), the therapeutic category "Vitamin/Vitamin Source" includes the following fat-soluble compounds:
alpha-tocopherol, beta-tocopherol, gamma-tocopherol, delta-tocopherol, tocopherol acetate, ergosterol, 1-alpha-hydroxycholecalciferol, vitamin D2, vitamin D3, alpha-carotene, beta-carotene, gamma-carotene, vitamin A, fursultiamine, methylolriboflavin, octotiamine, prosultiamine, riboflavine, vintiamol, dihydrovitamin K1, menadiol diacetate, menadiol dibutyrate, menadiol disulfate, menadiol, vitamin K1, vitamin K1 oxide, vitamins K2, and vitamin K-S(II).

Folic acid is also of this type, and although it is water-soluble at physiological pH, it can be formulated in the free acid form as a high-Kow compound.

A number of these contain chemical groups (amino, acidic, etc.) which can be titrated by addition of acid or base, and are useful in the present invention in some of the salt forms so created, particularly in cases where they retain their high partition coefficients even in the salt (charged) form. The use of alpha-tocopherol (vitamin E) as a dissolution/solubilization agent in the present invention is illustrated in Experiments 1 and 2.
**Amphiphilic derivatives of water-soluble vitamins and their salts**. Vitamins B, C, U, pantothenic acid, folic acid, and some of the menadione-related vitamins/provitamins in many of their various forms are considered as water-soluble vitamins, but when conjugated or complexed with hydrophobic moieties or multivalent ions may be put into amphiphilic forms having relatively high octanol-water partition coefficients, polar groups, and molecular weights between about 50 and 500. Again, such compounds can be of low toxicity and high benefit to risk ratio, making them of potential use as excipients and potentially as functional excipients. These compounds as well as salts thereof can be useful as dissolution/solubilization agents in the present invention, especially in modulating the polarity properties near the polar-apolar interfaces present in the structured fluid as described herein. According to the Merck Index (Eleventh Edition), the therapeutic category "Vitamin/Vitamin Source" includes the following water-soluble compounds:
acetiamine, benfotiamine, pantothenic acid, cetotiamine, cycothiamine, dexpanthenol, niacinamide, nicotinic acid, pyridoxal 5-phosphate, nicotinamide ascorbate, riboflavin phosphate, thiamine, folic acid, menadiol diphosphate, menadione sodium bisulfite, menadoxime, vitamin K5, vitamin K6, vitamin K6, and vitamin U.
Also, as mentioned above, folic acid is, over a wide pH range including physiological pH, water-soluble, as a salt.
Thus, of these compounds in which an amino or other basic group is listed, a high-Kow compound can often be created via a simple acid-base reaction with a hydrophobic group-containing acid such as a fatty acid (especially lauric, oleic, myristic, palmitic, stearic, or 2-ethylhexanoic acid), gentisic acid, low-solubility amino acid, benzoic acid, salicylic acid, acidic fat-soluble vitamin (such as riboflavine) or acidic component of an essential oil. Another approach is to react such an acid with another group on the vitamin such as a hydroxyl group to form a linkage such as an ester linkage, etc. Contrariwise, a water-soluble vitamin containing an acidic group can be reacted with a hydrophobic group-containing reactant such as stearylamine or riboflavine, for example, to create a high-Kow compound of potential use according to the present invention. The linkage of a palmitate chain to vitamin C yields ascorbyl palmitate, a compound (sometimes referred to as "a fat-soluble form of vitamin C") that is very useful in the present invention, as exemplified in Experiment 9.
**Low-water solubility amino acids and their salts**. Certain amino acids, in their zwitterionic form and/or in a salt form with a monovalent or multivalent ion, have polar groups, relatively high octanol-water partition coefficients, and molecular weights between about 50 and 500, and are very useful in the current invention. In the context of the present disclosure we take "low-solubility amino acid" to mean an amino acid which has a solubility in unbuffered water of less than about 4% (40 mg/ml). These include:
Cystine, tyrosine, tryptophan, leucine, isoleucine, phenylalanine, asparagine, aspartic acid, glutamic acid, and methionine.

The injectable product Nephramine, a parenteral nutrition product that has a long history of safe use, includes a number of amino acids each in quantities exceeding one gram, underscording the low toxicity of free amino acids-including low-solubility amino acids-even via injection.

Oligopeptides, with MWs less than about 500, can also be useful in this regard, as can multivalent ion salts of these amino acids and even the more soluble ones. Thus, other amino acids with higher solubilities than 4% can be used in the current invention, as multivalent ion salts or in oligomers with low-solubility amino acids.

As may be seen from Experiment 5, tryptophan is a desirable dissolution/solubilization agent. Indeed, tryptophan is especially useful as it partitions into the bilayer, and exhibits a high solubility in the bilayer, over a wide pH range and with a wide range of lipids and surfactants.

### Synergistic effects.

A number of synergistic effects occur when the above components are combined, particularly in the context of lyotropic liquid and especially lyotropic liquid crystal solubilization systems. For example, when a phosphatidylcholine-water mixture is converted to a non-lamellar phase by the addition of an essential oil or other compound, then the addition of gentisic acid salts such as gentisic acid ethanolamine, and related compounds as described herein, have a tendency to cause the mixture to revert back to lamellar, and in such a case the addition oftocopherol often provides a very effective and pharmaceutically acceptable path back to non-lamellar phases such as a reversed cubic phase. As another example, a number of gentisic acid salts are not soluble in phospholipid systems, but can be made so by the addition of relatively small amounts of essential oil; this is illustrated in Experiment 9. And for pharmaceutical compounds that contain both highly polar (e.g., charged) and aromatic and/or apolar groups, a judiciously designed mixture of phospholipid, aromatic essential oil, and charged salt such as a gentisic acid salt can provide for effective solubilization of the compound.

### Routes of Delivery.

The compositions of the present invention may be administered by any of a variety of means which are well known to those of skill in the art. These means include but are not limited to oral (e.g. via pills, tablets, lozenges, capsules, troches, syrups and suspensions, and the like) and non-oral routes (e.g. perenterally, intravenously, intraocularly, transdermally, via inhalation, and the like). The compositions of the present invention are particularly suited for internal (i.e. non-topical) administration.

### EXAMPLES

### Experiment 1.

The high-phosphatidylcholine lecithin Epikuron 200^{™} (from Lucas-Meyer), in the amount 0.351 grams, was combined with 0.371 gm of gentisic acid ethanolamine, 0.201 gm water, 0.156 gm glycerol, and 0.127 gm alpha-tocopherol, which upon mixing and equilibration formed a reversed cubic phase. This cubic phase was capable of solubilizing dantrolene sodium at body temperature. Thus, to 0.233 gm of this cubic phase was added 9 mg of dantrolene sodium, and after heating and then cooling to 37 C, the resulting cubic phase was clear, optically isotropic, and free of dantrolene crystals according to observation in the polarizing optical microscope and through crossed polarizing filters in a low-magnification (about 2x) optical setup.

### Experiment 2.

The high-phosphatidylcholine lecithin AEpikuron 200^{™}, in the amount 0.351 grams, was combined with 0.314 gm of gentisic acid ethanolamine, 0.199 gm water, 0.146 gm glycerol, and 0.125 gm alpha-tocopherol, which upon mixing and equilibration formed a reversed cubic phase. This cubic phase was capable of solubilizing the trans-platinum antineoplastic compound trans-[Pt(II)Cl₂(NH₃)(thiazole)] at a level of about 2.4%. Thus, 0.028 gm of this platinum compound was added to the cubic phase, and upon equilibration substantially all of the platinum compound was dissolved in the cubic phase. This cubic phase has almost identical composition with that in Experiment 1, underscoring the versatility of this composition in solubilizing a range of difficult drugs. Furthermore, a small increase in the gentisic acid ethanolamine concentration yields a lamellar phase, which could be useful in forming liposomes capable of incorporating paclitaxel and other drugs.

### Reference Experiment 3.

This example exemplifies the usefulness of combining alpha-tocopherol with phospholipid, essential oil, and water (or water plus glycerol) mixtures. Approximately 0.17 gm of Epikuron 200, 0.7 gm essential oil of sweet basil, 0.25 gm of water, and 0.25 gm of glycerol were mixed and the mixture centrifuged. The resulting 3-phase system featured only a small middle, liquid crystalline phase, and was mostly excess oil and excess water/glycerol. Then, upon the addition of about 0.33 gm of alpha-tocopherol, the liquid crystalline phase took up much more material so that at equilibrium, it was approximately equal in volume to the excess aqueous and oil phases. This liquid crystalline phase was capable of solubilizing paclitaxel to a level of at least 5 mg/ml.

### Reference Experiment 4.

The high-phosphatidylcholine lecithin Epikuron 200^{™}, in the amount 0.354 grams, was combined with 0.128 gm oflinalool, 0.379 gm of gentisic acid ethanolamine, 0.090 gm of essential oil of vanilla, and 0.273 gm water. The resulting equilibrated mixture was a cubic phase. Linalool has recently undergone extensive toxicity investigation, and has been shown to be of extremely low toxicity, not only by oral but also by intramuscular and intraperitoneal routes. Since gentisic acid ethanolamine has been used for decades in injectable products at a level of 100 mg per injection, in particular in a parenteral nutrition product, this mixture is composed of extremely benign components for drug-delivery.

### Experiment 5.

This example shows the effectiveness of the amino acid tryptophan in inducing reversed liquid crystalline phases in phosphatidylcholine systems. Epikuron 200, 0.549 grams, was combined with 0.166 gm of glycerol and 0.318 gm of water, which forms a lamellar phase in excess water, but the addition of only 0.023 gm of L-tryptophan resulted in a reversed cubic phase. The active alpha-lipoic acid can be solubilized to an appreciable extent in this cubic phase, for example.

### Experiment 6.

An antibiotic of Antex Biologics referred to as LH Syn 01, which had been problematic to solubilize with traditional means, in the amount of 2.034 grams, was dissolved in 6.028 grams of essential oil of ginger, together with 6 mg of BHT and 5 mg of BHA as antioxidants. To 7.852 grams of this solution were added 8.746 grams of the high-phosphatidylcholine lecithin Epikuron 200^{™}, and 4.687 grams of water. The mixture formed a reversed cubic phase on equilibration, with the LH syn 01 active fully solubilized.

### Experiment 7.

In this experiment, essential oils of ginger and sweet basil were combined to solubilize the bioactive compound ubiquinone (a coenzyme Q10) in a phospholipid-rich reversed liquid crystal. Coenzyme Q10, in the amount of 66 mg, was solubilized in a mixture of 0.408 gm oil of basil and 0.424 gm oil of ginger. To this was added 0.909 gm ofEpikuron 200 and 0.586 gm water. The Q10 was solubilized in the resulting reversed bicontinuous cubic phase liquid crystal.

### Experiment 8.

In this example the antibacterial compound 8-hydroxyquinoline was solubilized in a cubic phase. An amount of 62 mg of 8-hydroxyquinoline was dissolved in 0.311 gm oil of peppermint, to which were added 0.392 gm of Epikuron 200, 0.160 gm of glycerol, and 0.221 gm of water. The quinoline compound was solubilized in the resulting reversed bicontinuous cubic phase.

It should also be mentioned that 8-hydroxyquinoline is approved by the FDA for use as an inactive excipient in injectable formulations. Thus, in a cubic phase such as this, the 8-HQ could play the role of co-solubilizer, which by introducing amino groups into the bilayer could have a substantial enhancing effect on the solubilization of a number of actives.

### Experiment 9.

Epikuron 200, in the amount of 0.360 gm, 0.289 gm of ascorbyl palmitate, 0.141 gm of gentisic acid, 0.205 gm of aminocaproic acid, 0.106 gm of ethanolamine, and 0.461 gm water were combined and mixed thoroughly. The result was an opaque mix of undissolved crystals and one or more lipid-containing phases as determined by examination in a polarizing optical microscope that was equipped with phase contrast capabilities. An amount 0.517 gm of this mix was removed, and upon the addition of 0.109 gm of oil of ginger, all of the crystalline components dissolved and the result was a transparent reversed cubic liquid crystalline phase. Dantrolene sodium, in the amount of 3 mg, dissolved in this phase, which thus comprised a pharmaceutically-acceptable, lipid-based liquid crystalline solubilization matrix for this pharmaceutical compound.

### Experiment 10.

The local anesthetic bupivicaine, in the free base form, and in the amount of 0.096 grams, was combined with 0.376 gm linalool, 0.375 gm Pluronic P103, and 0.354 gm water. This formed a reversed cubic phase that, upon uptake of a small amount of water, can coexist with excess water. Both linalool (a component of a number of essential oils) and Pluronic P103 are of extremely low toxicity, making this cubic phase an attractive candidate as a depot delivery system for the local anesthetic. Increasing the duration of action of bupivicaine could be an important boon in the treatment of wounds including surgical wounds. The high octanol-water partition coefficient of bupivicaine should cause the bupivicaine to release slowly into body fluids.

### Reference Experiment 11.

Gum benzoin (obtained from Penta Chemicals, the "Siam" variety the active (as a functional excipient) was solubilized at the level of 1.0% in a cubic phase consisting of ylang-ylang oil, Pluronic P103, and water. The cubic phase can furthermore be dispersed as microparticles, and coated with a variety of coatings as described in published PCT Patent Application PCT/US98/18639 which can be formulated in oral or parenteral drug formulations for increased drug absorption.

## Claims

1. A composition comprising:
A) a reversed cubic liquid crystalline phase or a reversed hexagonal liquid crystalline phase structured fluid comprising:
i) a polar solvent,
ii) a lipid or a surfactant, and
iii) an essential oil or a dissolution/solubilization agent or both an essential oil and a dissolution/solubilization agent, said dissolution/solubilization agent being selected from the group consisting of
a. gentisic acid, benzoic acid, salicylic acid, N-alkylated amino acids, or a salt thereof;
b. a fat-soluble vitamin or a salt thereof;
c. amphiphilic derivatives of a water-soluble vitamin or a salt thereof;
d. 8-hydroxyquinoline; and
e. a low water-solubility amino acid or a salt thereof; and
B) a compound solubilized in said structured fluid, wherein said compound is otherwise less than 5 % weight soluble in soybean oil.

2. A composition comprising:
A) a reversed cubic liquid crystalline phase or a reversed hexagonal liquid crystalline phase structured fluid comprising:
i) a polar solvent,
ii) a lipid or a surfactant, and
iii) an essential oil or a dissolution/solubilization agent or both an essential oil and a dissolution/solubilization agent, said dissolution/solubilization agent having
a. at least one polar group in its molecular structure,
b. a molecular weight from about 50 to 500 Dalton and
c. an octanol-water partition coefficient greater than about 10; and
B) compound solubilized in said structured fluid, wherein said compound is otherwise less than 5 % weight soluble in soybean oil.

3. A composition comprising:
A) a reversed cubic liquid crystalline phase or a reversed hexagonal liquid crystalline phase structured fluid comprising:
i) a polar solvent,
ii) a lipid or a surfactant, and
iii) an essential oil or a dissolution/solubilization agent or both an essential oil and a dissolution/solubilization agent, said dissolution/solubilization agent being
selected from the group consisting of
a. gentisic acid, benzoic acid, salicylic acid, N-alkylated amino acids, or a salt thereof;
b. amphiphilic derivatives of a water-soluble vitamin or a salt thereof;
c. 8-hydroxyquinoline; and
d. a low water-solubility amino acid or a salt thereof; and
B) a compound solubilized in said structured fluid, wherein said compound is otherwise less than 5 % by weight soluble in soybean oil.

4. A composition according to claim 1 or 2, wherein the dissolution/solubilization agent is alpha-, beta-, gamma-, delta-tocopherol or tocopherol acetate.

5. An internally administerable solvent system comprising a reversed cubic liquid crystalline phase or a reversed hexagonal liquid crystalline phase structured fluid and in which a compound may be incorporated wherein said compound is otherwise less than 5 % by weight soluble in soybean oil said solvent system formed from
a. a polar solvent,
b. a lipid or a surfactant, and
c. an essential oil or a dissolution/solubilization agent or both an essential oil and a dissolution/solubilization agent, said solubilization agent being selected from
(i) gentisic acid, benzoic acid, salicylic acid, N-alkylated amino acids, or a salt thereof;
(ii) a fat-soluble vitamin or a salt thereof;
(iii) amphiphilic derivatives of a water-soluble vitamin or a salt thereof;
(iv) 8-hydroxyquinoline, and
(v) a low water-solubility amino acid or a salt thereof.

6. An internally administerable solvent system comprising a reversed cubic liquid crystalline phase or a reversed hexagonal liquid crystalline phase structured fluid and in which a compound may be incorporated wherein said compound is otherwise less than 5 % by weight soluble in soybean oil said solvent system formed from
a. a polar solvent,
b. a lipid or a surfactant, and
c. an essential oil or a dissolution/solubilization agent or both and essential oil and a dissolution/solubilization agent, said solubilization agent having
i. at least one polar group in its molecular structure,
ii. a molecular weight from about 50 to 500 Dalton and
iii. an octanol-water partition coefficient greater than about 10.

7. An internally administerable solvent system according to claim 5 wherein said essential oil or a dissolution/solubilization agent or both an essential oil and a dissolution/solubilization agent, said solubilization agent being selected from
(i) gentisic acid, benzoic acid, salicylic acid, N-alkylated amino acids, or a salt thereof
(ii) amphiphilic derivatives of a water-soluble vitamin or a salt thereof;
(iii) 8-hydroxyquinoline; and
(iv) a low water-solubility amino acid or a salt thereof.

8. An internally administerable solvent system according to claim 5 or 6 wherein the dissolution/solubilization agent is alpha-, beta-, gamma-, delta-tocopherol or tocopherol acetate.

9. A method for solubilizing a compound wherein said compound is otherwise I ess than 5 % by weight soluble in soybean oil comprising the steps of,
combining said compound with a solvent system comprising a reversed cubic liquid crystalline phase or a reversed hexagonal liquid crystalline phase structured fluid and in which a compound may be incorporated wherein said compound is otherwise less than 5 % by weight soluble in soybean oil said solvent system, wherein said structured fluid comprise(s)
a polar solvent,
a lipid or a surfactant, and
an essential oil or a dissolution/solubilization agent or both an essential oil and a dissolution/solubilization agent, said dissolution/solubilization agent being selected from the group consisting of
(i) gentisic acid, benzoic acid, salicylic acid, N-alkylated amino-acids, or a salt thereof;
(ii) a fat-soluble vitamin or a salt thereof;
(iii) amphiphilic derivatives of a water-soluble vitamin or a salt thereof;
(iv)8-hydroxyquinoline; and
(v) a low water-solubility amino acid or a salt thereof; and
allowing said compound to be solubilized into said solvent system.

10. A method for solubilizing a compound wherein said compound is otherwise I ess than 5 % by weight soluble in soybean oil comprising the steps of,
combining said compound with a solvent system comprising a reversed cubic liquid crystalline phase or a reversed hexagonal liquid crystalline phase structured fluid and in which a compound may be incorporated wherein said compound is otherwise less than 5 % by weight soluble in soybean oil said solvent system, wherein said structured fluid comprise(s)
a polar solvent,
a lipid or a surfactant, and
an essential oil or a dissolution/solubilization agent or both an essential oil and a dissolution/solubilization agent, said dissolution/solubilization agent being selected from the group agent having
a. at least one polar group in its molecular structure,
b. a molecular weight from about 50 to 500 Dalton and
c. an octanol-water partition coefficient greater than about 10;
and allowing said compound to be solubilized into said solvent system.

11. A method for solubilizing a compound according to claim 9, wherein said essential oil or a dissolution/solubilization agent or both an essential oil and a dissolution/solubilization agent, said dissolution/solubilization agent being selected from the group consisting of
(i) gentisic acid, benzoic acid, salicylic acid, N-alkylated amino acids, or a salt thereof;
(ii) amphiphilic derivatives of a water-soluble vitamin or a salt thereof;
(iii) 8-hydroxyquinoline; and
(iv)a low water-solubility amino acid or a salt thereof; and
allowing said compound to be incorporated into said solvent system.

12. The method according to anyone of claims 9 or 10, wherein the dissolution/solubilization agent is alpha-, beta-, gamma-, delta-tocopherol or tocopherol acetate.

## Patentansprüche

1. Zusammensetzung umfassend:
A) ein strukturiertes Fluid einer reversen kubischen flüssigkristallinen Phase oder einer reversen hexagonalen flüssigkristallinen Phase umfassend:
i) ein polares Lösungsmittel
ii) ein Lipid oder ein Tensid, und
iii) ein essentielles Öl oder ein Lösungs-/Solubilisierungsmittel oder beides, ein essentielles Öl und ein Lösungs-/Solubilisierungsmittel, das Lösungs-/Solubilisierungsmittel ist ausgewählt aus der Gruppe bestehend aus
a. Gentisinsäure, Benzoesäure, Salicylsäure, N-alkylierten Aminosäuren oder einem Salz davon;
b. ein fettlösliches Vitamin oder ein Salz davon;
c. amphiphile Derivate eines wasserlöslichen Vitamins oder einem Salz davon;
d. 8-Hydroxychinolin; und
e. eine Aminosäure mit geringer Wasserlöslichkeit oder einem Salz davon und
B) eine Verbindung die im strukturierten Fluid solubilisiert ist, wobei diese Verbindung ansonsten zu weniger als 5 % in Sojabohnenöl löslich ist.

2. Zusammensetzung umfassend:
A) strukturiertes Fluid einer reversen kubischen flüssigkristallinen Phase oder einer reversen hexagonalen flüssigkristallinen Phase umfassend
i) ein polares Lösungsmittel,
ii) ein Lipid oder ein Tensid,
iii) ein essentielles Öl oder ein Lösungs-/Solubilisierungsmittel oder beides, ein essentielles Öl und ein Lösungs-/Solubilisierungsmittel, das Lösungs-/Solubilisierungsmittel hat
a. mindestens ein polare Gruppe in seiner Molekülstruktur,
b. ein Molekulargewicht von ca. 50 bis 500 Dalton und
c. ein Octanol-Wasser-Verteilungskoeffizienten von größer als ca. 10; und
B) eine Verbindung solubilisiert in diesem strukturierten Fluid, wobei die Verbindung ansonsten zu weniger als 5 Gew.-% in Sojabohnenöl löslich ist.

3. Zusammensetzung umfassend:
A) ein strukturiertes Fluid einer reversen kubischen flüssigkristallinen Phase oder einer reversen hexagonalen flüssigkristallinen Phase umfassend:
i) ein polares Lösungsmittel
ii) ein Lipid oder ein Tensid, und
iv) ein essentielles Öl oder ein Lösungs-/Solubilisierungsmittel oder beides, ein essentielles Öl und ein Lösungs-/Solubilisierungsmittel, das Lösungs-/Solubilisierungsmittel ist ausgewählt aus der Gruppe bestehend aus
a. Gentisinsäure, Benzoesäure, Salicylsäure, N-alkylierten Aminosäuren oder einem Salz davon;
b. amphiphile Derivate eines wasserlöslichen Vitamins oder einem Salz davon;
c. 8-Hydroxychinolin; und
d. eine Aminosäure mit geringer Wasserlöslichkeit oder einem Salz davon und
C) eine Verbindung, die im strukturierten Fluid solubilisiert ist, wobei diese Verbindung ansonsten zu weniger als 5 % in Sojabohnenöl löslich ist.

4. Zusammensetzung gemäß Anspruch 1 oder 2, wobei das Lösungs-/Solubilisierungsmittel alpha-, beta-, gamma-, delta-Tocopherol oder Tocopherolacetat ist.

5. Ein innerlich verabreichbares Lösungsmittelsystem umfassend ein strukturiertes Fluid einer reversen kubischen flüssigkristallinen Phase oder einer reversen hexagonalen flüssigkristallinen Phase in die eine Verbindung integriert werden kann, wobei die Verbindung ansonsten zu weniger als 5 Gew.-% in Sojabohnenöl löslich ist, dieses Lösungsmittelsystem ist gebildet aus
a. einem polaren Lösungsmittel,
b. einem Lipid oder einem Tensid, und
c. einem essentiellen Öl oder einem Lösungs-/Solubilisierungsmittel oder beidem, einem essentiellen Öl und einem Lösungs-/Solubilisierungsmittel, das Solubilisierungsmittel ist ausgewählt aus
i) Gentisinsäure, Benzoesäure, Salicylsäure, N-alkylierten Aminosäuren oder einem Salz davon,
ii) einem fettlöslichen Vitamin oder einem Salz davon
iii) amphiphilen Derivaten eines wasserlöslichen Vitamins oder einem Salz davon;
iv) 8-Hydroxychinolin, und
v) einer Aminosäure mit geringer Wasserlöslichkeit oder einem Salz davon.

6. Ein innerlich verabreichbares Lösungsmittelsystem umfassend ein strukturiertes Fluid einer reversen kubischen flüssigkristallinen Phase oder einer reversen hexagonalen flüssigkristallinen Phase und in die eine Verbindung integriert werden kann, wobei die Verbindung ansonsten zu weniger als 5 Gew.-% löslich in Sojabohnenöl ist, dieses Lösungsmittelsystem ist gebildet aus
a. einem polaren Lösungsmittel,
b. einem Lipid oder einem Tensid, und
c. einem essentiellen Öl oder einem Lösungs-/Solubilisierungsmittel oder beidem, einem essentiellen Öl und einem Lösungs-/Solubilisierungsmittel, das Solubilisierungsmittel weist
i) mindestens eine polare Gruppe in seiner Molekülstruktur,
ii) ein Molekulargewicht von ca. 50 bis 500 Dalton und
iii) einen Octanol-Wasser-Verteilungskoeffizienten von größer ca. 10 auf.

7. Ein innerlich verabreichbares Lösungsmittelsystem gemäß Anspruch 5, wobei das essentielle Öl oder ein Lösungs-/Solubilisierungsmittel oder beides, ein essentielles Öl und ein Lösungs-/Solubilisierungsmittel, das Solubilisierungsmittel ausgewählt ist aus
i) Gentisinsäure, Benzoesäure, Salicylsäure, N-alkylierten Aminosäuren oder einem Salz davon,
ii) amphiphilen Derivaten eines wasserlöslichen Vitamins oder einem Salz davon;
iii) 8-Hydroxychinolin; und
iv) einer Aminosäure mit geringer Wasserlöslichkeit oder einem Salz davon.

8. Ein innerlich verabreichbares Lösungsmittelsystem gemäß Anspruch 5 oder 6, wobei das Lösungs-/Solubilisierungsmittel ein alpha-, beta-, gamma-, delta-Tocopherol oder Tocopherolacetat ist.

9. Verfahren zur Solubilisierung einer Verbindung, wobei die Verbindung ansonsten zu weniger als 5 Gew.-% in Sojabohnenöl löslich ist, umfassend die Schritte:
Kombinieren der Verbindung mit einem Lösungsmittelsystem umfassend ein strukturieres Fluid mit einer reversen kubischen flüssigkristallinen Phase oder einer reversen hexagonalen flüssigkristallinen Phase und in die eine Verbindung integriert werden kann, wobei diese Verbindung ansonsten zu weniger als 5 Gew.-% in Sojabohnenöl löslich ist, wobei das strukturierte Fluid umfasst
ein polares Lösungsmittel,
ein Lipid oder ein Tensid und
ein essentielles Öl oder ein Lösungs-/Solubilisierungsmittel oder beides, ein essentielle Öl und ein Lösungs-/Solubilisierungsmittel, das Lösungs-/Solubilisierungsmittel ist ausgewählt aus der Gruppe bestehend aus
i) Gentisinsäure, Benzoesäure, Salicylsäure, N-alkylierten Aminosäuren oder einem Salz davon;
ii) ein fettlösliches Vitamin oder einem Salz davon;
iii) amphiphile Derivate eines wasserlöslichen Vitamins oder einem Salz davon;
iv) 8-Hydroxychinolin; und
v) eine Aminosäure mit geringer Wasserlöslichkeit oder einem Salz davon und
Ermöglichen, dass diese Verbindung in diesem Lösungsmittelsystem solubilisiert wird.

10. Verfahren zur Solubilisierung einer Verbindung, wobei die Verbindung ansonsten zu weniger als 5 Gew.-% in Sojabohnenöl löslich ist, umfassend die Schritte:
Kombinieren der Verbindung mit einem Lösungsmittelsystem umfassend ein strukturieres Fluid mit einer reversen kubischen flüssigkristallinen Phase oder einer reversen hexagonalen flüssigkristallinen Phase und in die eine Verbindung integriert werden kann, wobei diese Verbindung ansonsten zu weniger als 5 Gew.-% in Sojabohnenöl löslich ist, wobei das strukturierte Fluid umfasst
ein polares Lösungsmittel,
ein Lipid oder ein Tensid und
ein essentielles Öl oder ein Lösungs-/Solubilisierungsmittel oder beides, ein essentielle Öl und ein Lösungs-/Solubilisierungsmittel, das Lösungs-/Solubilisierungsmittel ist ausgewählt aus der Gruppe bestehend aus
a. mindestens eine polare Gruppe in seiner Molekularstruktur
b. einem Molekulargewicht von ca. 50 bis 500 Dalton und
c. ein Octanol-Wasser-Verteilungskoeffizienten größer als ca. 10; und
Ermöglichen, dass diese Verbindung in diesem Lösungsmittelsystem solubilisiert wird.

11. Verfahren zum Solubilisieren einer Verbindung gemäß Anspruch 9, wobei das essentielle Öl oder eine Lösungs-/Solubilisierungsmittel oder beides, ein essentielles Öl und ein Lösungs-/Solubilisierungsmittel, dieses Lösungs-/Solubilisierungsmittel ist ausgewählt aus der Gruppe bestehend aus
i) Gentisinsäure, Benzoesäure, Salicylsäure, N-alkylierten Aminosäuren oder einem Salz davon;
ii) amphiphilen Derivaten eines wasserlöslichen Vitamins oder einem Salz davon;
iii) 8-Hydroxychinolin; und
iv) einer Aminosäure mit geringer Wasserlöslichkeit oder einem Salz davon und
Ermöglichen, dass diese Verbindung sich in dieses System integriert.

12. Verfahren gemäß einem der Ansprüche 9 oder 10, wobei das Lösungs/Sobulisierungsmittel alpha-, beta-, gamma-, delta-Tocopherol oder Tocopherolacetat ist.

## Revendications

1. Une composition comprenant :
A) un fluide structuré à phase cristalline liquide cubique inversée ou à phase cristalline liquide hexagonale inversée comprenant,
i) un solvant polaire,
ii) un lipide ou un surfactant, et
iii) une huile essentielle ou un agent de dissolution/solubilisation ou à la fois une huile essentielle et un agent de dissolution/solubilisation, ledit agent de dissolution/solubilisation étant choisi dans le groupe constitué par :
a. l'acide gentisique, l'acide benzoique, l'acide salicylique, les amino acides N-alkylés ou un de leurs sels ;
b. une vitamine soluble dans la graisse ou un sel de celle-ci ;
c. des dérivés amphiphiliques d'une vitamine soluble dans l'eau ou un sel de ceux-ci ;
d. de la 8-hydroxyquinoline ; et
e. un amino acide de faible solubilité dans l'eau ou un sel de celui-ci ; et
B) un composé solubilisé dans ledit fluide structuré, dans lequel ledit composé est en outre soluble à moins de 5% en poids dans l'huile de soja.

2. Une composition comprenant :
A) un fluide structuré à phase cristalline liquide cubique inversée ou à phase cristalline liquide hexagonale inversée comprenant,
i) un solvant polaire,
ii) un lipide ou un surfactant, et
iii) une huile essentielle ou un agent de dissolution/solubilisation ou à la fois une huile essentielle et un agent de dissolution/solubilisation, ledit agent de dissolution/solubilisation ayant
a. au moins un groupe polaire dans sa structure moléculaire,
b. un poids moléculaire d'environ 50 à 500 Dalton et
c. un coefficient de séparation octanol-eau supérieur à environ 10 ; et
B) un composé solubilisé dans ledit fluide structuré, dans lequel ledit composé est en outre soluble à moins de 5% en poids dans l'huile de soja.

3. Une composition comprenant :
A) un fluide structuré à phase cristalline liquide cubique inversée ou à phase cristalline liquide hexagonale inversée comprenant,
i) un solvant polaire,
ii) un lipide ou un surfactant, et
iii) une huile essentielle ou un agent de dissolution/solubilisation ou à la fois une huile essentielle et un agent de dissolution/solubilisation, ledit agent de dissolution/ssolubilisation étant choisi dans le groupe constitué par :
a. l'acide gentisique, l'acide benzoique, l'acide salicylique, les amino acides N-alkylés ou un de leurs sels ;
b. des dérivés amphiphiliques d'une vitamine soluble dans l'eau ou un sel de ceux-ci ;
c. de la 8-hydroxyquinoline ; et
d. un amino acide de faible solubilité dans l'eau ou un sel de celui-ci ; et
B) un composé solubilisé dans ledit fluide structuré, dans lequel ledit composé est en outre soluble à moins de 5% en poids dans l'huile de soja.

4. Une composition selon la revendication 1 ou 2, dans laquelle l'agent de dissolution/solubilisation est de l'alpha-, bêta-, gamma-, delta- tocophérol ou de l'acétate de tocophérol.

5. Un système de solvant administrable par voie interne comprenant un fluide structuré à phase cristalline liquide cubique ou à phase cristaline hexagonale et dans lequel peut être incorporé un composé, ledit composé étant en outre soluble à moins de 5% en poids dans l'huile de soja, ledit système de solvant étant formé de :
a) un solvant polaire,
b) un lipide ou un surfactant, et
c) une huile essentielle ou un agent de dissolution/solubilisation ou à la fois une huile essentielle et un agent de dissolution/solubilisation, ledit agent de dissolution/solubilisation étant choisi dans le groupe constitué par :
i) l'acide gentisique, l'acide benzoique, l'acide salicylique, les amino acides N-alkylés ou un de leurs sels ;
ii) une vitamine soluble dans la graisse ou un sel de celle-ci ;
iii) des dérivés amphiphiliques d'une vitamine soluble dans l'eau ou un sel de ceux-ci ;
iv) de la 8-hydroxyquinoline ; et
v) un amino acide de faible solubilité dans l'eau ou un sel de celui-ci ;

6. Un système de solvant administrable par voie interne comprenant un fluide structuré à phase cristalline liquide cubique ou à phase cristaline hexagonale et dans lequel peut être incorporé un composé, ledit composé étant en outre soluble à moins de 5% en poids dans l'huile de soja, ledit système de solvant étant formé de :
a) un solvant polaire,
b) un lipide ou un surfactant, et
c) une huile essentielle ou un agent de dissolution / solubilisation ou à la fois une huile essentielle et un agent de dissolution / solubilisation, ledit agent de dissolution / solubilisation ayant
i) an moins un groupe polaire dans sa structure moléculaire,
ii) un poids moléculaire d'environ 50 à 500 Dalton et
iii) un coefficient de séparation octanol-eau supérieur à environ 10.

7. Un système de solvant administrable par voie interne selon la revendication 5, dans lequel ladite huile essentielle ou ledit agent de dissolution / solubilisation ou à la fois une huile essentielle et un agent de dissolution / solubilisation, ledit agent de solubilisation étant choisi dans le groupe constitué par :
i) l'acide gentisique, l'acide benzoique, l'acide salicylique, les amino acides N - alkylés ou un de leurs sels ;
ii) des dérivés amphiphiliques d'une vitamine soluble dans l'eau ou un sel de ceux-ci,
iii) de la 8 - hydroxyquinoline ; et
iv) un amino acide à faible seolubilité dans l'eau ou un sel de celui-ci.

8. Un système de solvant selon la revendication 5 ou 6 dans lequel l'agent de dissolution/solubilisation est de l'alpha-, bêta-, gamma-, delta-tocophérol ou de l'acétate de tocophérol.

9. Un procédé pour solubiliser un composé dans lequel ledit composé est en outre soluble à moins de 5% en poids dans l'huile de soja comprenant les étapes de combiner ledit composé avec un système de solvant comprenant un fluide structuré à phase cristalline liquide cubique inversée ou à phase cristalline liquide hexagonale et dans lequel peut-être incorporé un composé, ledit composé étant en outre soluble à moins de 5% en poids dans l'huile de soja, ledit système de solvant étant formé de :
- un solvant polaire,
- un lipide ou une surfactant, et une huile essentielle ou un agent de dissolution / solubilisation ou à la fois une huile essentielle et un agent de dissolution / solubilisation, ledit agent de dissolution / solubilisation étant choisi dans le groupe constitué par :
i) l'acide gentisique, l'acide benzoique, l'acide salicylique, les amino acides N - alkylés ou un de leurs sels ;
ii) une vitamine soluble dans la graisse ou un sel de celle-ci
iii) des dérivés amphiliques d'une vitamine soluble dans l'eau ou un sel de ceux-ci ;
iv) de la 8 - hydroxyquinnoline ; et
v) un amino acide de faible solubilité dans l'eau ou un sel de celui-ci et laisser ledit composé se solubiliser dans ledit système de solvant.

10. Un procédé pour solubiliser un composé dans lequel ledit composé est en outre soluble à moins de 5% en poids dans l'huile de soja comprenant les étapes de combiner ledit composé avec un système de solvant comprenant un fluide structuré à phase cristalline liquide cubique inversée ou à phase cristalline liquide hexagonale et dans lequel peut-être incorporé un composé, ledit composé étant en outre soluble à moins de 5% en poids dans l'huile de soja, ledit système de solvant étant formé de :
- un solvant polaire,
- un lipide ou un surfactant, et une huile essentielle ou un agent de dissolution / solubilisation ou à la fois une huile essentielle et un agent de dissolution / solubilisation, ledit agent de dissolution / solubilisation étant choisi dans le groupe d'agents ayant :
a) au moins un groupe polaire dans sa structure moléculaire,
b) un poids moléculaire d'environ 50 à 500 Dalton et
c) un coefficient de séparation octanol-eau supérieur à environ 10 et laisser ledit composé se solubiliser dans ledit système de solvant.

11. Un procédé pour solubiliser un composé selon la revendication 9, dans lequel ladite huile essentielle ou ledit agent de dissolution / solubilisation ou à la fois une huile essentielle et un agent de dissolution / solubilisation, ledit agent de dissolution / solubilisation étant choisi dans le groupe constitué par :
i) l'acide gentisique, l'acide benzoique, l'acide salicytique, les amino acides N - alkylés ou un de leurs sels ;
ii) des dérivés amphiphiliques d'une vitamine soluble dans l'eau ou un sel de ceux-ci ;
iii) de la 8 - hydroxyquinoline ; et
iv) un amino acide de faible solubilité dans l'eau ou un sel de celui-ci ; et laisser ledit composé s'incorporer dans ledit système de solvant.

12. Le procédé selon l'une quelconque des revendication 9 à 10, dans lequel l'agent de dissolution/solubilisation est de l'alpha-, bêta-, gamma-, delta-tocophérol ou de l'acétate de tocophérol.
